# EUROPEAN PATENT SPECIFICATION

(11) **EP 4 370 675 B1**
(45) Date of publication and mention of the grant of the patent: **01.10.2025**
(21) Application number: 22762200.8
(22) Date of filing: 10.08.2022
(51) Int. Cl.: C12N 15/10, C12Q 1/6837

(54) **METHODS, COMPOSITIONS AND SYSTEMS FOR IDENTIFYING ANTIGEN-BINDING MOLECULES**
VERFAHREN, ZUSAMMENSETZUNGEN UND SYSTEME ZUR IDENTIFIZIERUNG VON ANTIGENBINDENDEN MOLEKÜLEN
PROCEDES, COMPOSITIONS ET SYSTEMES D'IDENTIFICATION DE MOLECULES DE LIAISON A L'ANTIGENE

(30) Priority: 12.08.2021 US 202163232386 P
(43) Date of publication of application: 22.05.2024
(73) Proprietor: 10X Genomics, Inc., Pleasanton, CA 94588-3260 (US)
(72) Inventor: GALONSKA, Christina, 113 63 Stockholm (SE); STOECKIUS, Marlon, 113 63 Stockholm (SE)
(74) Representative: Vossius & Partner Patentanwälte Rechtsanwälte mbB
(86) International application number: PCT/US2022/039956
(87) International publication number: WO 2023/018799

(56) References cited:
- WO-A1-2020/123309
- WO-A1-2020/243579
- WO-A1-2021/091611
- WO-A1-2021/097255
- WO-A1-2021/133849
- WO-A2-2020/047004
- WO-A2-2020/123301
- WO-A2-2021/092433
- LIU YANG ET AL: "Spatial-CITE-seq: spatially resolved high-plex protein and whole transcriptome co-mapping", BIORXIV, 2 April 2022 (2022-04-02), XP093001136, Retrieved from the Internet <URL:https://www.biorxiv.org/content/10.1101/2022.04.01.486788v1.full.pdf> [retrieved on 20221123], DOI: 10.1101/2022.04.01.486788
- UENO SHINGO ET AL: "cDNA Display: Rapid Stabilization of mRNA Display", ANTIBODY-DRUG CONJUGATES; IN: METHODS IN MOLECULAR BIOLOGY; ISSN 1064-3745; VOL. 263; [METHODS IN MOLECULAR BIOLOGY; ISSN 1064-3745], HUMANA PRESS, US, vol. 805, 1 January 2012 (2012-01-01), pages 113 - 135, XP009190355, ISBN: 978-1-62703-541-5, DOI: 10.1007/978-1-61779-379-0_8
- RUPAK DOSHI ET AL: "In vitro nanobody discovery for integral membrane protein targets", SCIENTIFIC REPORTS, vol. 4, 24 October 2014 (2014-10-24), pages 6760, XP055334236, DOI: 10.1038/srep06760
- HUANG YICHAO ET AL: "RNA Display Methods for the Discovery of Bioactive Macrocycles", CHEMICAL REVIEWS, vol. 119, no. 17, 5 November 2018 (2018-11-05), US, pages 10360 - 10391, XP093001580, ISSN: 0009-2665, DOI: 10.1021/acs.chemrev.8b00430
- MARLON STOECKIUS ET AL: "Simultaneous epitope and transcriptome measurement in single cells", NATURE METHODS, vol. 14, no. 9, 31 July 2017 (2017-07-31), New York, pages 865 - 868, XP055556042, ISSN: 1548-7091, DOI: 10.1038/nmeth.4380
- MARLON STOECKIUS ET AL: "Cell Hashing with barcoded antibodies enables multiplexing and doublet detection for single cell genomics", GENOME BIOLOGY, vol. 19, no. 1, 1 December 2018 (2018-12-01), XP055702284, DOI: 10.1186/s13059-018-1603-1
- LIU YANG ET AL: "High-Spatial-Resolution Multi-Omics Sequencing via Deterministic Barcoding in Tissue", CELL, ELSEVIER, AMSTERDAM NL, vol. 183, no. 6, 13 November 2020 (2020-11-13), pages 1665, XP086400370, ISSN: 0092-8674, [retrieved on 20201113], DOI: 10.1016/J.CELL.2020.10.026

## Description

### TECHNICAL FIELD OF THE INVENTION

This invention relates to methods including single-chain antibody nucleic acid libraries and methods of displaying single-chain antibodies and screening single-chain antibodies on a spatial array.

### BACKGROUND

Cells within a tissue have differences in cell morphology and/or function due to varied analyte levels (e.g., gene and/or protein expression) within the different cells. The specific position of a cell within a tissue (e.g., the cell's position relative to neighboring cells or the cell's position relative to the tissue microenvironment) can affect, e.g., the cell's morphology, differentiation, fate, viability, proliferation, behavior, signaling, and cross-talk with other cells in the tissue.

Spatial heterogeneity has been previously studied using techniques that typically provide data for a handful of analytes in the context of intact tissue or a portion of a tissue (e.g., tissue section), or provide significant analyte data from individual, single cells, but fails to provide information regarding the position of the single cells from the originating biological sample (e.g., tissue).

Therapeutic antibodies are an essential class of drugs which are widely used in the treatment of cancer, autoimmunity, inflammatory diseases, and/or for drug delivery to target antigens. Current antibody therapeutics discovery workflows typically involve (1) target assessment and validation, (2) screening preparation, (3) hit generation, screening, and lead selection. These classic workflows are limited in that a target of interest must generally be known prior to screening. Since there are few known targets, many antibody campaigns target the same limited number of existing drug targets, while novel targets are seldom explored. Furthermore, the screening process generally utilizes *in vitro* models or cell lines which do not accurately reflect the *in vivo* context, where targets can be embedded within a complex tissue environment. Furthermore, the screening processes are not typically suitable for identifying antibody therapeutics for personalized medicine. Therefore, a need remains for methods, compositions, and systems for improved antibody therapeutics discovery.

### SUMMARY

The invention is set out in the appended set of claims.

The present disclosure features methods of generating an antigen-binding molecule/nucleic acid conjugate library, methods of displaying (e.g., ribosomal display, mRNA/cDNA display) antigen-binding molecules/nucleic acid libraries, methods of identifying antigen-binding molecules that selectively bind to antigens in a test biological sample and determining the location of the antigen in the test biological sample, and compositions and kits thereof.

Therapeutic antibodies are an essential class of drugs which are widely used in the treatment of cancer, autoimmunity, and inflammatory diseases, and/or for drug delivery to target antigens. Conventional antibodies (e.g., IgG) are large in size (e.g., about 150 kDa) and comprise two heavy and two light chains. Smaller, single-chain, antigen-binding molecules (e.g., about 15 kDa) can be attractive alternatives to conventional antibodies. In some examples, single-chain antibodies include scFv single-chain antibodies. In some examples, single-chain antibodies include single variable domain on a heavy chain (VHH) antibodies, also known as nanobodies. These single-chain antibodies are generally more stable, easier to generate large libraries, and easier to screen than conventional antibodies. Additionally, the single-chain antibodies can be screened via various display techniques. In some examples, the display technique is ribosomal display. In some examples, the display technique is mRNA/cDNA display. These screening techniques can also be performed in combination with a spatial array (e.g., an array comprising a plurality of capture probes, where a capture probe includes a spatial barcode and a capture domain). For example, single-chain antibodies can be displayed in an in vitro system, screened against a healthy biological sample (e.g., healthy tissue section), and the non-binding antibodies can be collected (e.g., washed off) the biological sample and screened against a diseased biological sample (e.g., a tumor sample). Non-binding antibodies can be removed from the diseased biological sample (e.g., washed off) and the remaining single-chain antibodies bound to an antigen present in the diseased can be captured on a spatial array as described herein and identified.

Provided herein are methods of generating an antigen-binding molecule/nucleic acid conjugate library, where an antigen-binding molecule/nucleic acid conjugate of the antigen-binding molecule/nucleic acid conjugate library includes an antigen binding molecule linked to a nucleic acid encoding the antigen-binding molecule, the method including: (a) providing a plurality of in vitro transcribed mRNAs, where an mRNA of the plurality of in vitro transcribed mRNAs includes in a 5' to 3' direction: (i) an analyte capture sequence that hybridizes to a capture domain of a capture probe, (ii) a sequence encoding the antigen-binding molecule, (iii) a primer binding sequence, (iv) a universal domain attached to a donor oligonucleotide, where the donor oligonucleotide includes a linker having a nucleotide sequence and a protein translation inhibitor; and (b) in vitro translating the plurality of in vitro transcribed mRNAs, thereby generating the antigen-binding molecule/nucleic acid conjugate library antigen-binding molecule mRNA.

In some embodiments, the method includes a prior step of attaching the donor oligonucleotide to the universal domain via ligation. In some embodiments, the ligation includes ligation with a splint oligonucleotide. In some embodiments, the splint oligonucleotide is substantially complementary to the linker of the donor oligonucleotide, or a portion thereof. In some embodiments, the splint oligonucleotide is substantially complementary to the universal domain, or a portion thereof.

In some embodiments, the ligating includes Y-ligation. In some embodiments, the linker of the donor oligonucleotide is substantially complementary to the universal domain, or a portion thereof. In some embodiments, the method includes ligating the 5' end of the donor oligonucleotide to the 3' end of the universal domain.

In some embodiments, the ligating includes a ligase. In some embodiments, the ligase is selected from the group consisting of T4 DNA ligase, T4 RNA ligase, PBCV-1 ligase and a Chlorella DNA ligase.

In some embodiments, the ligating includes ultra-violet (UV) light cross-linking. In some embodiments, the linker of the donor oligonucleotide is substantially complementary to the universal domain, or a portion thereof. In some embodiments, the 5' end of the linker includes a UV light photoreactive moiety. In some embodiments, the UV light photoreactive moiety includes psoralen.

In some embodiments, the linker of the donor oligonucleotide is substantially complementary to the universal domain, or a portion thereof.

In some embodiments, the protein translation inhibitor is non-covalently attached to the mRNA. In some embodiments, the protein translation inhibitor is covalently attached to the mRNA. In some embodiments, the protein translation inhibitor molecule is puromycin.

In some embodiments, the antigen-binding molecule includes a single-chain antigen-binding molecule. In some embodiments, the single-chain antigen binding molecule includes a single-chain variable fragment (scFv). In some embodiments, the single-chain antigen binding molecule includes a nanobody.

In some embodiments, the method includes generating a cDNA complementary to the mRNA to produce a double stranded DNA/RNA duplex.

In some embodiments, generating the cDNA includes reverse transcribing the mRNA. In some embodiments, reverse transcription includes use of a primer that hybridizes to the universal domain of the mRNA.

In some embodiments, the protein translation inhibitor does not include a stop codon.

Also provided herein are methods of identifying an antigen-binding molecule that selectively binds to an antigen in a biological sample, the method including: (a) providing an array including a plurality of capture probes, where a capture probe of the plurality includes (i) a spatial barcode and (ii) a capture domain; (b) contacting the biological sample with members of an antigen-binding molecule/nucleic acid conjugate library, where an antigen-binding molecule/nucleic acid conjugate of the library includes an antigen binding molecule linked to a nucleic acid encoding the antigen-binding molecule, and where the nucleic acid includes a cDNA/RNA duplex , where the RNA strand of the duplex includes: (i) a sequence complementary to the analyte capture sequence which binds a capture domain of a capture probe, (ii) a sequence encoding an antigen-binding molecule, (iii) a primer binding sequence, and (iv) a universal domain attached to a donor oligonucleotide, where the donor oligonucleotide includes a linker having a nucleotide sequence and a protein translation inhibitor, (c) removing one or more unbound antigen-binding molecule/nucleic acid conjugates from the biological sample; and (d) capturing one or more cDNAs of the antigen-binding molecule/nucleic acid conjugates onto one or more capture probes of the array, where an analyte capture sequence of the cDNA binds to the capture domain of the capture probe.

In some embodiments, the method includes determining (i) the sequence of the spatial barcode or a complement thereof, and (ii) all or a portion of the sequence of the cDNA, or a complement thereof. In some embodiments, the method includes using the determined sequences of (i) the sequence of the spatial barcode or a complement thereof and (ii) all or a portion of the sequence of the cDNA or a complement thereof to identify an antigen-binding molecule that selectively binds to an antigen in the biological sample and determine the location of the antigen in the biological sample. In some embodiments, the determining includes sequencing.

In some embodiments, step (c) is performed before (a), after (a), or after (b).

In some embodiments, the members of the antigen-binding molecule/nucleic acid conjugate library were selected by a prior step of contacting a control biological sample with the antigen-binding molecule/nucleic acid conjugate library, and selecting members of the library that did not bind to the control biological sample.

In some embodiments, the control biological sample is a healthy biological sample. In some embodiments, the biological sample includes one or more healthy regions and one or more disease regions. In some embodiments, the biological sample is a diseased biological sample. In some embodiments, the diseased biological sample is a tumorous biological sample.

In some embodiments, the method includes releasing the cDNA from the antigen-binding molecule/nucleic acid conjugate prior to the capturing step. In some embodiments, the releasing includes an RNase. In some embodiments, the RNase is selected from the group consisting of RNase A, RNase C, RNase H, or RNase I. In some embodiments, the releasing includes heat.

In some embodiments, the method includes permeabilizing the biological sample, the test biological sample, or both. In some embodiments, the method includes imaging the biological sample. In some embodiments, the method includes staining the biological sample. In some embodiments, the staining includes hematoxylin and eosin (H&E) staining.

In some embodiments, the array includes one or more features. In some embodiments, the one or more features includes a bead.

In some embodiments, the capture probe includes any one or more of: a cleavage domain, a unique molecular identifier (UMI), one or more functional domain, and combinations thereof.

In some embodiments, the antigen-binding molecule is a single-chain antigen-binding molecule. In some embodiments, the single-chain antigen-binding molecule includes a single-chain variable fragment (scFv). In some embodiments, the single-chain antigen-binding molecule includes a nanobody.

In some embodiments, the cDNA complementary to the nucleic acid of the antigen-binding molecule/nucleic acid conjugate is generated by reverse transcription.

In some embodiments, the method includes capturing a nucleic acid analyte or derivative thereof from the biological sample, where the nucleic acid analyte binds to the capture domain of the capture probe. In some embodiments, the nucleic acid analyte, or derivative thereof, includes DNA. In some embodiments, the nucleic acid analyte, or derivative thereof, includes RNA (e.g., mRNA).

In some embodiments, the method includes: (a) contacting a first probe and a second probe with the biological sample, where the first probe and the second probe each include one or more sequences that are substantially complementary to sequences of an analyte, and where the second probe includes a capture probe capture domain that is complementary to all or a portion of the capture domain; (b) hybridizing the first probe and the second probe to the analyte; (c) generating a ligation product by ligating the first probe and the second probe; (d) releasing the ligation product from the analyte; (e) hybridizing the ligation product to the capture domain; and (g) determining (i) all or part of the sequence of the ligation product bound to the capture domain, or a complement thereof, and (ii) all of the spatial barcode, or a complement thereof, and using the determined sequences of (i) and (ii) to identify the location of the analyte in the biological sample.

In some embodiments, the method includes identifying the antigen bound by the antigen-binding molecule/nucleic acid complex. In some embodiments, the identifying includes mass spectrometry. In some embodiments, the identifying includes determining differential gene expression patterns between the control biological sample and the test biological sample. In some embodiments, the identifying includes determining differential gene expression patterns within the biological sample.

In some embodiments, the biological sample is disposed on the spatial array including the plurality of capture probes. In some embodiments, the biological sample is disposed on the substrate.

In some embodiments, the method includes aligning the substrate with the spatial array, such that at least a portion of the biological sample is aligned with at least a portion of the spatial array.

Also provided herein are compositions including: an in vitro transcribed mRNA in a 5' to 3' direction: (i) a sequence complementary to an analyte capture sequence that hybridized to a capture domain of a capture probe, (ii) a sequence encoding an antigen-binding molecule, (iii) a primer binding sequence, and (iv) a universal sequence.

In some embodiments, the method includes a donor oligonucleotide attached to the universal sequence of the mRNA. In some embodiments, the donor oligonucleotide includes a linker having a sequence and a protein translation inhibitor. In some embodiments, the protein translation inhibitor includes a translation inhibitor molecule including puromycin.

In some embodiments, the donor oligonucleotide is attached the universal sequence of the mRNA with a splint oligonucleotide. In some embodiments, the splint oligonucleotide includes a substantially complementary sequence to the universal domain and a substantially complementary sequence to the linker of the donor oligonucleotide.

In some embodiments, the protein translation inhibitor does not include a stop codon.

Also provided herein are composition including: an antigen-binding molecule/nucleic acid conjugate including: (i) a sequence complementary to an analyte capture sequence, (ii) a sequence encoding an antigen-binding molecule, (iii) a primer binding sequence, (iv) a constant sequence; (v) a protein translation inhibitor molecule; and (vi) the antigen-binding molecule.

In some embodiments, the antigen-binding molecule includes a single-chain antigen binding molecule. In some embodiments, the single-chain antigen binding molecule includes an scFv. In some embodiments, the single-chain antigen binding molecule includes a nanobody.

In some embodiments, the composition includes a cDNA hybridized to the nucleic acid of the antigen-binding molecule/nucleic acid conjugate. In some embodiments, the cDNA is generated by reverse transcription.

Also provided herein are compositions including: (a) an array including a plurality of capture probes, where a capture probe includes: (i) a spatial barcode; and (ii) a capture domain; and b) a cDNA including in the 5' to 3' direction: (i) a sequence complementary to a universal domain; (ii) a sequence complementary to a primer binding site; (iii) a sequence complementary to a sequence encoding an antigen-binding molecule; and (iv) an analyte capture sequence, where the analyte capture sequence is bound to the capture domain of the capture probe.

Also provided herein are compositions including: an array including a plurality of capture probes, where a capture probe includes: (i) a spatial barcode; and (ii) a capture domain; and b) an extended cDNA product including in the 5' to 3' direction: (i) a sequence complementary to a universal domain; (ii) a sequence complementary to a primer binding site; (iii) a sequence complementary to a sequence encoding an antigen-binding molecule; (iv) an analyte capture sequence, where the analyte capture sequence is bound to the capture domain of the capture probe; (v) a sequence complementary the capture probe; and c) an extended capture probe including a sequence complementary to the cDNA using the cDNA as an extension template.

In some embodiments, the capture probe includes one or more functional domains, a UMI, a cleavage domain, and combinations thereof.

Where values are described in terms of ranges, it should be understood that the description includes the disclosure of all possible sub-ranges within such ranges, as well as specific numerical values that fall within such ranges irrespective of whether a specific numerical value or specific sub-range is expressly stated.

The term "each," when used in reference to a collection of items, is intended to identify an individual item in the collection but does not necessarily refer to every item in the collection, unless expressly stated otherwise, or unless the context of the usage clearly indicates otherwise.

Various embodiments of the features of this disclosure are described herein. However, it should be understood that such embodiments are provided merely by way of example, and numerous variations, changes, and substitutions can occur to those skilled in the art.

### DESCRIPTION OF DRAWINGS

The following drawings illustrate certain embodiments of the features and advantages of this disclosure. Like reference symbols in the drawings indicate like elements.
**FIG. 1** is a schematic diagram showing an example of a barcoded capture probe, as described herein.
**FIGs. 2A-E** show an exemplary single-chain antibody nucleic acid library (**FIG. 2A**) and an exemplary method of generating a display library via in vitro transcription of the single-chain antibody nucleic acid library (**FIGs. 2B-E**).
**FIGs. 3A-B**show exemplary antigen-binding molecule/nucleic acid conjugate members binding to an antigen in a biological sample (**FIG. 3A**) and removal of non-binding antigen-binding molecule/nucleic acid conjugate members (**FIG. 3B**).
**FIGs. 4A-B**show non-binders from **FIG. 3B** binding to a diseased biological sample (**FIG. 4A**) and removal of non-binding antigen-binding molecule/nucleic acid conjugate members (**FIG. 4B**).
**FIGs. 5A-B**show a capture probe hybridized to the analyte capture sequence of the cDNA of the antigen-binding molecule/nucleic acid conjugate members (**FIG. 5A**) and extension of the 3' end of the capture probe and the 3' end of the cDNA (**FIG. 5B**).
**FIGs. 6A-B**show extension of the 3' end of the capture probe and the 3' end of the cDNA (**FIG. 6A**) and the resulting nucleic acid library in **FIG. 6B**.

### DETAILED DESCRIPTION

The present disclosure features methods of generating an antigen-binding molecule/nucleic acid conjugate library, methods of displaying (e.g., ribosomal display, mRNA/cDNA display) antigen-binding molecules/nucleic acid libraries, methods of identifying antigen-binding molecules that selectively bind to antigens in a test biological sample, and determining the location of the antigen in the test biological sample, and compositions and kits thereof.

Therapeutic antibodies are an essential class of drugs which are widely used in the treatment of cancer, autoimmunity, and inflammatory diseases, and/or for drug delivery to target antigens. Conventional antibodies (e.g., IgG) are large in size (e.g., about 150 kDa) and comprise two heavy and two light chains. Smaller, single-chain, antigen-binding molecules (e.g., about 15 kDa) can be attractive alternatives to conventional antibodies. In some examples, single-chain antibodies include scFv single-chain antibodies. In some examples, single-chain antibodies include VHH antibodies (e.g., nanobodies). These single-chain antibodies are generally more stable, easier to generate large libraries, and easier to screen than conventional antibodies. Additionally, the single-chain antibodies can be screened via various display techniques. In some examples, the display technique is ribosomal display. In some examples, the display technique is mRNA/cDNA display. These screening techniques can also be performed in combination with a spatial array (e.g., an array comprising a plurality of capture probes, where a capture probe comprises a spatial barcode and a capture domain). For example, single-chain antibodies can be displayed in an in vitro system, screened against a healthy biological sample (e.g., healthy tissue section), and the non-binding antibodies can be collected (e.g., washed off) the biological sample and screened against a diseased biological sample (e.g., a tumor sample). Non-binding antibodies can be removed from the diseased biological sample (e.g., washed off) and the remaining single-chain antibodies bound to an antigen present in the diseased can be captured on a spatial array as described herein and identified.

Spatial analysis methodologies and compositions described herein can provide a vast amount of analyte and/or expression data for a variety of analytes within a biological sample at high spatial resolution, while retaining native spatial context. Spatial analysis methods and compositions can include, e.g., the use of a capture probe including a spatial barcode (e.g., a nucleic acid sequence that provides information as to the location or position of an analyte within a cell or a tissue sample (e.g., mammalian cell or a mammalian tissue sample)) and a capture domain that is capable of binding to an analyte (e.g., a protein and/or a nucleic acid) produced by and/or present in a cell. Spatial analysis methods and compositions can also include the use of a capture probe having a capture domain that captures an intermediate agent for indirect detection of an analyte. For example, the intermediate agent can include a nucleic acid sequence (e.g., a barcode) associated with the intermediate agent. Detection of the intermediate agent is therefore indicative of the analyte in the cell or tissue sample.

Non-limiting aspects of spatial analysis methodologies and compositions are described in U.S. Patent Nos. 10,774,374, 10,724,078, 10,480,022, 10,059,990, 10,041,949, 10,002,316, 9,879,313, 9,783,841, 9,727,810, 9,593,365, 8,951,726, 8,604,182, 7,709,198, U.S. Patent Application Publication Nos. 2020/239946, 2020/080136, 2020/0277663, 2020/024641, 2019/330617, 2019/264268, 2020/256867, 2020/224244, 2019/194709, 2019/161796, 2019/085383, 2019/055594, 2018/216161, 2018/051322, 2018/0245142, 2017/241911, 2017/089811, 2017/067096, 2017/029875, 2017/0016053, 2016/108458, 2015/000854, 2013/171621, WO 2018/091676, WO 2020/176788, Rodriques et al., Science 363(6434):1463-1467, 2019; Lee et al., Nat. Protoc. 10(3):442-458, 2015; Trejo et al., PLoS ONE 14(2):e0212031, 2019; Chen et al., Science 348(6233):aaa6090, 2015; Gao et al., BMC Biol. 15:50, 2017; and Gupta et al., Nature Biotechnol. 36:1197-1202, 2018; the Visium Spatial Gene Expression Reagent Kits User Guide (e.g., Rev C, dated June 2020), and/or the Visium Spatial Tissue Optimization Reagent Kits User Guide (e.g., Rev C, dated July 2020), both of which are available at the 10x Genomics Support Documentation website, and can be used herein in any combination. Ueno and Nemoto, cDNA display: rapid stabilization of mRNA display, Methods Mol Biol., 805: 113-35 (2012) and Doshi, R., et al., In vitro nanobody discovery for integral membrane protein targets. Sci Rep, 4, 6760 (2014) disclose methods of generating an antigen-binding molecule/nucleic acid conjugate library, wherein an antigen-binding molecule/nucleic acid conjugate of the antigen-binding molecule/nucleic acid conjugate library comprises an antigen binding molecule linked to a nucleic acid encoding the antigen-binding molecule, the method comprising: a) providing a plurality of in vitro transcribed mRNAs, wherein an mRNA of the plurality of in vitro transcribed mRNAs comprises in a 5' to 3' direction:(i) a sequence encoding the antigen-binding molecule,(ii) a primer binding sequence, (iii) a universal domain attached to a donor oligonucleotide, wherein the donor oligonucleotide comprises a linker having a nucleotide sequence and a protein translation inhibitor; and b) in vitro translating the plurality of in vitro transcribed mRNAs, thereby generating the antigen-binding molecule/nucleic acid conjugate library antigen-binding molecule mRNA. The method further comprises reverse transcribing the mRNA to generate a cDNA complementary to the mRNA to produce a double-stranded DNA/RNA duplex. Huang, Y., et al., RNA Display Methods for the Discovery of Bioactive Macrocycles, Chem. Rev., 119, 10360-10391 (2018) discloses a method of generating an antigen-binding molecule/nucleic acid conjugate library, wherein an antigen-binding molecule/nucleic acid conjugate of the antigen-binding molecule molecule/nucleic acid conjugate library comprises an antigen binding molecule linked to a nucleic acid encoding the antigen-binding molecule, the method comprising providing a plurality of in vitro transcribed mRNAs, wherein an mRNA of the plurality of in vitro transcribed mRNAs comprises in a 5' to 3' direction at least a sequence encoding the antigen-binding molecule and a universal domain attached to a donor molecule, wherein the donor oligonucleotide comprises a linker having a nucleotide sequence and a protein translation inhibitor (puromycin); and in vitro translating the plurality of in vitro transcribed mRNAs, thereby generating the antigen-binding molecule/nucleic acid conjugate library antigen-binding molecule mRNA. The oligonucleotide is ligated to the universal domain via ligation with a splint oligonucleotide, using Y-ligation, UV crosslinking or a TRAP system. The method may further comprise generating a cDNA complementary to the mRNA to produce a double-stranded DNA/RNA complex. Further non-limiting aspects of spatial analysis methodologies and compositions are described herein.

Some general terminology that may be used in this disclosure can be found in Section (I)(b) of WO 2020/176788 and/or U.S. Patent Application Publication No. 2020/0277663. Typically, a "barcode" is a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. For the purpose of this disclosure, an "analyte" can include any biological substance, structure, moiety, or component to be analyzed. The term "target" can similarly refer to an analyte of interest.

Analytes can be broadly classified into one of two groups: nucleic acid analytes, and non-nucleic acid analytes. Examples of non-nucleic acid analytes include, but are not limited to, lipids, carbohydrates, peptides, proteins, glycoproteins (N-linked or O-linked), lipoproteins, phosphoproteins, specific phosphorylated or acetylated variants of proteins, amidation variants of proteins, hydroxylation variants of proteins, methylation variants of proteins, ubiquitylation variants of proteins, sulfation variants of proteins, viral proteins (e.g., viral capsid, viral envelope, viral coat, viral accessory, viral glycoproteins, viral spike, etc.), extracellular and intracellular proteins, antibodies, and antigen binding fragments. In some embodiments, the analyte(s) can be localized to subcellular location(s), including, for example, organelles, e.g., mitochondria, Golgi apparatus, endoplasmic reticulum, chloroplasts, endocytic vesicles, exocytic vesicles, vacuoles, lysosomes, etc. In some embodiments, analyte(s) can be peptides or proteins, including without limitation antibodies and enzymes. Additional examples of analytes can be found in Section (I)(c) of WO 2020/176788 and/or U.S. Patent Application Publication No. 2020/0277663. In some embodiments, an analyte can be detected indirectly, such as through detection of an intermediate agent, for example, a ligation product or an analyte capture agent (e.g., an oligonucleotide-conjugated antibody), such as those described herein.

A "biological sample" is typically obtained from the subject for analysis using any of a variety of techniques including, but not limited to, biopsy, surgery, and laser capture microscopy (LCM), and generally includes cells and/or other biological material from the subject. In some embodiments, a biological sample can be a tissue section. In some embodiments, a biological sample can be a fixed and/or stained biological sample (e.g., a fixed and/or stained tissue section). Non-limiting examples of stains include histological stains (e.g., hematoxylin and/or eosin) and immunological stains (e.g., fluorescent stains). In some embodiments, a biological sample (e.g., a fixed and/or stained biological sample) can be imaged. Biological samples are also described in Section (I)(d) of WO 2020/176788 and/or U.S. Patent Application Publication No. 2020/0277663.

In some embodiments, a biological sample is permeabilized with one or more permeabilization reagents. For example, permeabilization of a biological sample can facilitate analyte capture. Exemplary permeabilization agents and conditions are described in Section (I)(d)(ii)(13) or the Exemplary Embodiments Section of WO 2020/176788 and/or U.S. Patent Application Publication No. 2020/0277663.

Array-based spatial analysis methods involve the transfer of one or more analytes from a biological sample to an array of features on a substrate, where each feature is associated with a unique spatial location on the array. Subsequent analysis of the transferred analytes includes determining the identity of the analytes and the spatial location of the analytes within the biological sample. The spatial location of an analyte within the biological sample is determined based on the feature to which the analyte is bound (e.g., directly or indirectly) on the array, and the feature's relative spatial location within the array.

A "capture probe" refers to any molecule capable of capturing (directly or indirectly) and/or labelling an analyte (e.g., an analyte of interest) in a biological sample. In some embodiments, the capture probe is a nucleic acid or a polypeptide. In some embodiments, the capture probe includes a barcode (e.g., a spatial barcode and/or a unique molecular identifier (UMI)) and a capture domain). In some embodiments, a capture probe can include a cleavage domain and/or a functional domain (e.g., a primer-binding site, such as for next-generation sequencing (NGS)). See, e.g., Section (II)(b) (e.g., subsections (i)-(vi)) of WO 2020/176788 and/or U.S. Patent Application Publication No. 2020/0277663. Generation of capture probes can be achieved by any appropriate method, including those described in Section (II)(d)(ii) of WO 2020/176788 and/or U.S. Patent Application Publication No. 2020/0277663.

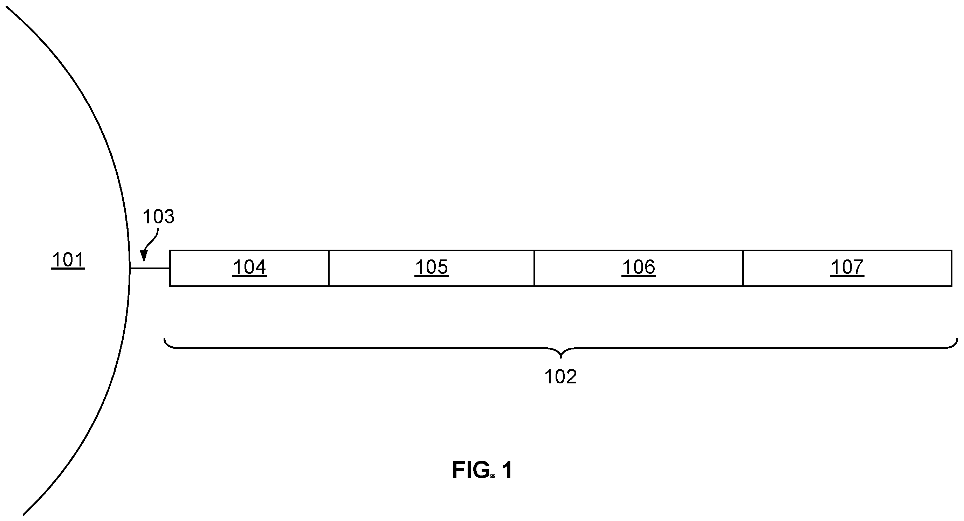

**FIG. 1** is a schematic diagram showing an exemplary capture probe, as described herein. As shown, the capture probe 102 is optionally coupled to a feature 101 by a cleavage domain 103, such as a disulfide linker. The capture probe can include a functional sequence 104 that is useful for subsequent processing. The functional sequence 104 can include all or a part of sequencer specific flow cell attachment sequence (e.g., a P5 or P7 sequence), all or a part of a sequencing primer sequence, (e.g., a R1 primer binding site, a R2 primer binding site), or combinations thereof. The capture probe can also include a spatial barcode 105. The capture probe can also include a unique molecular identifier (UMI) sequence 106. While FIG. 1 shows the spatial barcode 105 as being located upstream (5') of UMI sequence 106, it is to be understood that capture probes wherein UMI sequence 106 is located upstream (5') of the spatial barcode 105 is also suitable for use in any of the methods described herein. The capture probe can also include a capture domain 107 to facilitate capture of a target analyte. In some embodiments, the capture probe comprises one or more additional functional sequences that can be located, for example between the spatial barcode 105 and the UMI sequence 106, between the UMI sequence 106 and the capture domain 107, or following the capture domain 107. The capture domain can have a sequence complementary to a sequence of a nucleic acid analyte. The capture domain can have a sequence complementary to a capture handle sequence present in an analyte capture agent. The capture domain can have a sequence complementary to a splint oligonucleotide. Such splint oligonucleotide, in addition to having a sequence complementary to a capture domain of a capture probe, can have a sequence of a nucleic acid analyte, and/or a capture handle sequence described herein.

The functional sequences can generally be selected for compatibility with any of a variety of different sequencing systems, e.g., Ion Torrent Proton or PGM, Illumina sequencing instruments, PacBio, Oxford Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Ion Torrent Proton or PGM sequencing, Illumina sequencing, PacBio SMRT sequencing, and Oxford Nanopore sequencing. Further, in some embodiments, functional sequences can be selected for compatibility with other sequencing systems, including non-commercialized sequencing systems.

In some embodiments, the spatial barcode 105 and functional sequences 104 is common to all of the probes attached to a given feature (e.g., a bead, a well, a spot on an array). In some embodiments, the UMI sequence 106 of a capture probe attached to a given feature is different from the UMI sequence of a different capture probe attached to the given feature.

In some embodiments, more than one analyte type (e.g., nucleic acids and proteins) from a biological sample can be detected (e.g., simultaneously or sequentially) using any appropriate multiplexing technique, such as those described in Section (IV) of WO 2020/176788 and/or U.S. Patent Application Publication No. 2020/0277663.

In some embodiments, detection of one or more analytes (e.g., protein analytes) can be performed using one or more analyte capture agents. As used herein, an "analyte capture agent" refers to an agent that interacts with an analyte (e.g., an analyte in a biological sample) and with a capture probe (e.g., a capture probe attached to a substrate or a feature) to identify the analyte. In some embodiments, the analyte capture agent includes: (i) an analyte binding moiety (e.g., that binds to an analyte), for example, an antibody or antigen-binding fragment thereof; (ii) analyte binding moiety barcode; and (iii) an analyte capture sequence. As used herein, the term "analyte binding moiety barcode" refers to a barcode that is associated with or otherwise identifies the analyte binding moiety. As used herein, the term "analyte capture sequence" refers to a region or moiety configured to hybridize to, bind to, couple to, or otherwise interact with a capture domain of a capture probe. In some cases, an analyte binding moiety barcode (or portion thereof) may be able to be removed (e.g., cleaved) from the analyte capture agent. Additional description of analyte capture agents can be found in Section (II)(b)(ix) of WO 2020/176788 and/or Section (II)(b)(viii) U.S. Patent Application Publication No. 2020/0277663.

There are at least two methods to associate a spatial barcode with one or more neighboring cells, such that the spatial barcode identifies the one or more cells, and/or contents of the one or more cells, as associated with a particular spatial location. One method is to promote analytes or analyte proxies (e.g., intermediate agents) out of a cell and towards a spatially-barcoded array (e.g., including spatially-barcoded capture probes). Another method is to cleave spatially-barcoded capture probes from an array and promote the spatially-barcoded capture probes towards and/or into or onto the biological sample.

In some cases, capture probes may be configured to prime, replicate, and consequently yield optionally barcoded extension products from a template (e.g., a DNA or RNA template, such as an analyte or an intermediate agent (e.g., a ligation product or an analyte capture agent), or a portion thereof), or derivatives thereof (see, e.g., Section (II)(b)(vii) of WO 2020/176788 and/or U.S. Patent Application Publication No. 2020/0277663 regarding extended capture probes). In some cases, capture probes may be configured to form ligation products with a template (e.g., a DNA or RNA template, such as an analyte or an intermediate agent, or portion thereof), thereby creating ligations products that serve as proxies for a template.

As used herein, an "extended capture probe" refers to a capture probe having additional nucleotides added to the terminus (e.g., 3' or 5' end) of the capture probe thereby extending the overall length of the capture probe. For example, an "extended 3' end" indicates additional nucleotides were added to the most 3' nucleotide of the capture probe to extend the length of the capture probe, for example, by polymerization reactions used to extend nucleic acid molecules including templated polymerization catalyzed by a polymerase (e.g., a DNA polymerase or a reverse transcriptase). In some embodiments, extending the capture probe includes adding to a 3' end of a capture probe a nucleic acid sequence that is complementary to a nucleic acid sequence of an analyte or intermediate agent specifically bound to the capture domain of the capture probe. In some embodiments, the capture probe is extended using reverse transcription. In some embodiments, the capture probe is extended using one or more DNA polymerases. The extended capture probes include the sequence of the capture probe and the sequence of the spatial barcode of the capture probe.

In some embodiments, extended capture probes are amplified (e.g., in bulk solution or on the array) to yield quantities that are sufficient for downstream analysis, e.g., via DNA sequencing. In some embodiments, extended capture probes (e.g., DNA molecules) act as templates for an amplification reaction (e.g., a polymerase chain reaction).

Additional variants of spatial analysis methods, including in some embodiments, an imaging step, are described in Section (II)(a) of WO 2020/176788 and/or U.S. Patent Application Publication No. 2020/0277663. Analysis of captured analytes (and/or intermediate agents or portions thereof), for example, including sample removal, extension of capture probes, sequencing (e.g., of a cleaved extended capture probe and/or a cDNA molecule complementary to an extended capture probe), sequencing on the array (e.g., using, for example, in situ hybridization or in situ ligation approaches), temporal analysis, and/or proximity capture, is described in Section (II)(g) of WO 2020/176788 and/or U.S. Patent Application Publication No. 2020/0277663. Some quality control measures are described in Section (II)(h) of WO 2020/176788 and/or U.S. Patent Application Publication No. 2020/0277663.

Spatial information can provide information of biological and/or medical importance. For example, the methods and compositions described herein can allow for: identification of one or more biomarkers (e.g., diagnostic, prognostic, and/or for determination of efficacy of a treatment) of a disease or disorder; identification of a candidate drug target for treatment of a disease or disorder; identification (e.g., diagnosis) of a subject as having a disease or disorder; identification of stage and/or prognosis of a disease or disorder in a subject; identification of a subject as having an increased likelihood of developing a disease or disorder; monitoring of progression of a disease or disorder in a subject; determination of efficacy of a treatment of a disease or disorder in a subject; identification of a patient subpopulation for which a treatment is effective for a disease or disorder; modification of a treatment of a subject with a disease or disorder; selection of a subject for participation in a clinical trial; and/or selection of a treatment for a subject with a disease or disorder.

Spatial information can provide information of biological importance. For example, the methods and compositions described herein can allow for: identification of transcriptome and/or proteome expression profiles (e.g., in healthy and/or diseased tissue); identification of multiple analyte types in close proximity (e.g., nearest neighbor analysis); determination of up- and/or down-regulated genes and/or proteins in diseased tissue; characterization of tumor microenvironments; characterization of tumor immune responses; characterization of cells types and their co-localization in tissue; and identification of genetic variants within tissues (e.g., based on gene and/or protein expression profiles associated with specific disease or disorder biomarkers).

Typically, for spatial array-based methods, a substrate functions as a support for direct or indirect attachment of capture probes to features of the array. A "feature" is an entity that acts as a support or repository for various molecular entities used in spatial analysis. In some embodiments, some or all of the features in an array are functionalized for analyte capture. Exemplary substrates are described in Section (II)(c) of WO 2020/176788 and/or U.S. Patent Application Publication No. 2020/0277663. Exemplary features and geometric attributes of an array can be found in Sections (II)(d)(i), (II)(d)(iii), and (II)(d)(iv) of WO 2020/176788 and/or U.S. Patent Application Publication No. 2020/0277663.

Generally, analytes and/or intermediate agents (or portions thereof) can be captured when contacting a biological sample with a substrate including capture probes (e.g., a substrate with capture probes embedded, spotted, printed, fabricated on the substrate, or a substrate with features (e.g., beads, wells) comprising capture probes). As used herein, "contact," "contacted," and/or "contacting," a biological sample with a substrate refers to any contact (e.g., direct or indirect) such that capture probes can interact (e.g., bind covalently or non-covalently (e.g., hybridize)) with analytes from the biological sample. Capture can be achieved actively (e.g., using electrophoresis) or passively (e.g., using diffusion). Analyte capture is further described in Section (II)(e) of WO 2020/176788 and/or U.S. Patent Application Publication No. 2020/0277663.

In some cases, spatial analysis can be performed by attaching and/or introducing a molecule (e.g., a peptide, a lipid, or a nucleic acid molecule) having a barcode (e.g., a spatial barcode) to a biological sample (e.g., to a cell in a biological sample). In some embodiments, a plurality of molecules (e.g., a plurality of nucleic acid molecules) having a plurality of barcodes (e.g., a plurality of spatial barcodes) are introduced to a biological sample (e.g., to a plurality of cells in a biological sample) for use in spatial analysis. In some embodiments, after attaching and/or introducing a molecule having a barcode to a biological sample, the biological sample can be physically separated (e.g., dissociated) into single cells or cell groups for analysis. Some such methods of spatial analysis are described in Section (III) of WO 2020/176788 and/or U.S. Patent Application Publication No. 2020/0277663.

In some cases, spatial analysis can be performed by detecting multiple oligonucleotides that hybridize to an analyte. In some instances, for example, spatial analysis can be performed using RNA-templated ligation (RTL). Methods of RTL have been described previously. See, e.g., Credle et al., Nucleic Acids Res. 2017 Aug 21;45(14):e128. Typically, RTL includes hybridization of two oligonucleotides to adjacent sequences on an analyte (e.g., an RNA molecule, such as an mRNA molecule). In some instances, the oligonucleotides are DNA molecules. In some instances, one of the oligonucleotides includes at least two ribonucleic acid bases at the 3' end and/or the other oligonucleotide includes a phosphorylated nucleotide at the 5' end. In some instances, one of the two oligonucleotides includes a capture domain (e.g., a poly(A) sequence, a non-homopolymeric sequence). After hybridization to the analyte, a ligase (e.g., SplintR ligase) ligates the two oligonucleotides together, creating a ligation product. In some instances, the two oligonucleotides hybridize to sequences that are not adjacent to one another. For example, hybridization of the two oligonucleotides creates a gap between the hybridized oligonucleotides. In some instances, a polymerase (e.g., a DNA polymerase) can extend one of the oligonucleotides prior to ligation. After ligation, the ligation product is released from the analyte. In some instances, the ligation product is released using an endonuclease (e.g., RNAse H). The released ligation product can then be captured by capture probes (e.g., instead of direct capture of an analyte) on an array, optionally amplified, and sequenced, thus determining the location and optionally the abundance of the analyte in the biological sample.

During analysis of spatial information, sequence information for a spatial barcode associated with an analyte is obtained, and the sequence information can be used to provide information about the spatial distribution of the analyte in the biological sample. Various methods can be used to obtain the spatial information. In some embodiments, specific capture probes and the analytes they capture are associated with specific locations in an array of features on a substrate. For example, specific spatial barcodes can be associated with specific array locations prior to array fabrication, and the sequences of the spatial barcodes can be stored (e.g., in a database) along with specific array location information, so that each spatial barcode uniquely maps to a particular array location.

Alternatively, specific spatial barcodes can be deposited at predetermined locations in an array of features during fabrication such that at each location, only one type of spatial barcode is present so that spatial barcodes are uniquely associated with a single feature of the array. Where necessary, the arrays can be decoded using any of the methods described herein so that spatial barcodes are uniquely associated with array feature locations, and this mapping can be stored as described above.

When sequence information is obtained for capture probes and/or analytes during analysis of spatial information, the locations of the capture probes and/or analytes can be determined by referring to the stored information that uniquely associates each spatial barcode with an array feature location. In this manner, specific capture probes and captured analytes are associated with specific locations in the array of features. Each array feature location represents a position relative to a coordinate reference point (e.g., an array location, a fiducial marker) for the array. Accordingly, each feature location has an "address" or location in the coordinate space of the array.

Some exemplary spatial analysis workflows are described in the Exemplary Embodiments section of WO 2020/176788 and/or U.S. Patent Application Publication No. 2020/0277663. See, for example, the Exemplary embodiment starting with "In some non-limiting examples of the workflows described herein, the sample can be immersed..." of WO 2020/176788 and/or U.S. Patent Application Publication No. 2020/0277663. See also, e.g., the Visium Spatial Gene Expression Reagent Kits User Guide (e.g., Rev C, dated June 2020), and/or the Visium Spatial Tissue Optimization Reagent Kits User Guide (e.g., Rev C, dated July 2020).

In some embodiments, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

The systems can optionally include a control unit that includes one or more electronic processors, an input interface, an output interface (such as a display), and a storage unit (e.g., a solid state storage medium such as, but not limited to, a magnetic, optical, or other solid state, persistent, writeable and/or re-writeable storage medium). The control unit can optionally be connected to one or more remote devices via a network. The control unit (and components thereof) can generally perform any of the steps and functions described herein. Where the system is connected to a remote device, the remote device (or devices) can perform any of the steps or features described herein. The systems can optionally include one or more detectors (e.g., CCD, CMOS) used to capture images. The systems can also optionally include one or more light sources (e.g., LED-based, diode-based, lasers) for illuminating a sample, a substrate with features, analytes from a biological sample captured on a substrate, and various control and calibration media.

The systems can optionally include software instructions encoded and/or implemented in one or more of tangible storage media and hardware components such as application specific integrated circuits. The software instructions, when executed by a control unit (and in particular, an electronic processor) or an integrated circuit, can cause the control unit, integrated circuit, or other component executing the software instructions to perform any of the method steps or functions described herein.

In some cases, the systems described herein can detect (e.g., register an image) the biological sample on the array. Exemplary methods to detect the biological sample on an array are described in WO 2021/102003 A1and/or U.S. Patent Application Publication No. US 2021/0150707 A1.

Prior to transferring analytes from the biological sample to the array of features on the substrate, the biological sample can be aligned with the array. Alignment of a biological sample and an array of features including capture probes can facilitate spatial analysis, which can be used to detect differences in analyte presence and/or level within different positions in the biological sample, for example, to generate a three-dimensional map of the analyte presence and/or level. Exemplary methods to generate a two- and/or three-dimensional map of the analyte presence and/or level are described in WO 2021/067514 A1and spatial analysis methods are generally described in WO 2020/061108 and/or U.S. Patent Application Publication No. US 2021/0155982 A1.

In some cases, a map of analyte presence and/or level can be aligned to an image of a biological sample using one or more fiducial markers, e.g., objects placed in the field of view of an imaging system which appear in the image produced, as described in the *Substrate Attributes* Section, *Control Slide for Imaging* Section of WO 2020/123320, WO 2021/102005 A1, and/or U.S. Patent Application Publication No. US 2021/0158522 A1. Fiducial markers can be used as a point of reference or measurement scale for alignment (e.g., to align a sample and an array, to align two substrates, to determine a location of a sample or array on a substrate relative to a fiducial marker) and/or for quantitative measurements of sizes and/or distances.

### Methods and Compositions Including Antigen-Binding Molecule Libraries and Uses Thereof

The present disclosure features methods of generating an antigen-binding molecule/nucleic acid conjugate library, methods of displaying (e.g., ribosomal display, mRNA/cDNA display) antigen-binding molecules/nucleic acid libraries, methods of identifying antigen-binding molecules that selectively bind to antigens in a test biological sample and determining the location of the antigen in the test biological sample, and compositions and kits thereof.

Therapeutic antibodies are an essential class of drugs which are widely used in the treatment of cancer, autoimmunity, and inflammatory diseases, and/or for drug delivery to target antigens. Conventional antibodies (e.g., IgG) are large in size (e.g., about 150 kDa) and includes two heavy and two light chains. Additionally, in conventional antibody screening a target of interest generally needs to be known. However, since few targets are known, most antibody screens repeatedly test against the same existing drug targets and novel targets are seldom explored. Conventional antibody screening generally occurs *in vitro* on selected cell lines and may not recapitulate the *in vivo* context, where the target cells/peptides are embedded within a complex tissue environment and often the screening process is time intensive and offers little room for customization (e.g. by using patient biopsies).

Smaller, single-chain, antigen-binding molecules (e.g., about 15 kDa) can be attractive alternatives to conventional antibodies. In some examples, single-chain antibodies include scFv single-chain antibodies. In some examples, single-chain antibodies include VHH antibodies (e.g., nanobodies). These single-chain antibodies are generally more stable, easier to generate large libraries, and easier to screen than conventional antibodies. For example, single-chain antibody libraries can be generated with a diversity (e.g., different single-chain antibodies) of about 1 x 10⁶ to about 1 x 10¹⁵, about 1 x 10⁷ to about 1 x 10¹⁴, about 1 x 10⁸ to about 1 x 10¹³, or about 1 x 10⁹ to about 1 x 10¹². The methods described herein can also identify novel targets in the context of a complex tissue environment in a rapid manner and can be screened against healthy and/or diseased tissues.

Additionally, the single-chain antibodies can be screened via various display techniques. In some examples, the display technique is ribosomal display. In some examples, the display technique is mRNA/cDNA display. In some examples, the display technique is yeast display. These screening techniques can also be performed in combination with a spatial array (e.g., an array including a plurality of capture probes, where a capture probe includes a spatial barcode and a capture domain). For example, single-chain antibodies can be displayed in an *in vitro* system, screened against a healthy biological sample (e.g., healthy tissue section), and the non-binding antibodies can be collected (e.g., washed off) the biological sample and screened against a diseased biological sample (e.g., a tumor sample). Non-binding antibodies can be removed from the diseased biological sample (e.g., washed off) and the remaining single-chain antibodies bound to an antigen present in the diseased biological sample can be captured on a spatial array as described herein and identified.

Thus, the present disclosure features methods of generating an antigen-binding molecule/nucleic acid conjugate library, where an antigen-binding molecule/nucleic acid conjugate of the antigen-binding molecule/nucleic acid conjugate library includes an antigen-binding molecule linked to a nucleic acid encoding the antigen-binding molecule, the method including: a) providing a plurality of mRNAs, wherein an mRNA of the plurality of mRNAs includes in a 5' to 3' direction: (i) an analyte capture sequence which binds a capture domain of a capture probe, or a reverse complement thereof, (ii) a sequence encoding the antigen-binding molecule, (iii) a primer binding sequence, (iv) a universal domain attached to a donor oligonucleotide, where the donor oligonucleotide includes a linker having a nucleotide sequence and a protein translation inhibitor; b) *in vitro* translating the plurality of mRNAs, thereby generating the antigen-binding molecule/nucleic acid conjugate library antigen-binding molecule mRNA.

The present disclosure also features methods of screening antigen-binding molecule/nucleic acid conjugates in a biological sample (e.g., a healthy biological sample, a diseased biological sample). Thus, provided herein are methods of identifying an antigen-binding molecule that selectively binds to an antigen in a biological sample, the method including: a) contacting the biological sample with members of an antigen-binding molecule/nucleic acid conjugate library, wherein an antigen-binding molecule/nucleic acid conjugate of the library includes an antigen-binding molecule linked to a nucleic acid encoding the antigen-binding molecule, and where the nucleic acid includes a cDNA/RNA duplex, wherein the RNA strand of the duplex includes: i) a sequence complementary to the analyte capture sequence which binds (e.g., hybridizes) a capture domain of a capture probe, ii) a sequence encoding an antigen-binding molecule, iii) a primer binding sequence, and iv) a universal domain attached to a donor oligonucleotide, where the donor oligonucleotide includes a linker having a nucleotide sequence and a protein translation inhibitor, b) removing one or more unbound antigen-binding molecule/nucleic acid conjugates from the biological sample c) contacting a biological sample with an array including a plurality of capture probes, where a capture probe of the plurality includes (i) a spatial barcode and (ii) a capture domain; and d) capturing one or more cDNAs of the antigen-binding molecule/nucleic acid conjugates onto one or more capture probes of the array, where an analyte capture sequence of the cDNA binds to the capture domain of the capture probe.

### Single-chain Antigen-Binding Molecule

As used herein a "single-chain antigen-binding molecule" includes single-chain antibodies, single-chain antigen-binding fragments, and single-chain antigen-binding fusion proteins. In some embodiments, a single-chain antigen-binding molecule is a nanobody. In some embodiments, a single-chain antigen-binding molecule is a single-chain variable fragment (scFv) fusion protein.

A "nanobody" (Nb), as used herein, can refer to a single domain antibody. Nanobodies can include VHH antibodies (single variable domain on a heavy chain antibody) and can be derived from naturally occurring heavy chain antibody. Nanobodies can be derived from heavy chain only antibodies as found in camelids (Hamers-Casterman et al., Naturally occurring antibodies devoid of light chains, Nature, 363, 446-448 (1993)). "Camelids" include old world camelids (e.g., *Camelus bactrianus, Camelus dromedarius*) and new world camelids, such as llamas (e.g., *Lama paccos, Lama glama, Lama guanicoe,* and *Lama vicugna*). The single variable domain heavy chain antibody can be referred to herein as a "nanobody" or a VHH antibody. It is noted that the term "nanobody," as used herein in its broadest sense, is not limited to a specific biological source or to a specific method of preparation. For example, the nanobodies hereof, and their associated nucleotide sequences, can generally be obtained: (1) by isolating the VHH domain of a naturally occurring heavy chain antibody; (2) by expression of a nucleotide sequence encoding a naturally occurring VHH domain; (3) by "humanization" of a naturally occurring VHH domain or by expression of a nucleic acid encoding such a humanized VHH domain; (4) by "camelization" of a naturally occurring VH domain from any animal species, and, in particular, from a mammalian species, such as from a human being, or by expression of a nucleic acid encoding such a camelized VH domain; (5) by "camelization" of a "domain antibody" or "Dab" as described in the art, or by expression of a nucleic acid encoding such a camelized VH domain; (6) by using synthetic or semi-synthetic techniques for preparing proteins, polypeptides or other amino acid sequences known per se; (7) by preparing a nucleic acid encoding a nanobody using techniques for nucleic acid synthesis known per se, followed by expression of the nucleic acid thus obtained; and/or (8) by any combination of one or more of the foregoing.

The small size and unique biophysical properties of nanobodies provide alternatives to conventional antibody fragments since they can recognize uncommon or hidden epitopes and can bind into cavities or active sites of protein targets.

Single-chain variable fragment antibodies (scFvs) are single-chain antigen-binding molecules (e.g., single-chain antigen-binding fusion proteins) that can be used in any of the methods described herein. Generally, an scFv is a fusion protein of the variable region of the heavy (V_{H}) and the light chain (V_{L}) of immunoglobulins, thus lacking the F_{C} domain, and are usually connected with a short peptide linker of about 10 to about 25 amino acids. Generally, the linker can include a series of variants generated to optimize binding affinity and stability. Variations of scFv molecules have been engineered including paired scFvs that bind to one another through complementary regions to form bivalent molecules (diabodies), complementary scFvs themselves produced as a single chain (tandem scFvs or tascFvs), and bispecific tandem scFvs (bis-scFvs), as well as others.

Thus, in some embodiments, the antigen-binding molecule includes a single-chain antigen-binding molecule. In some embodiments, the single-chain antigen binding molecule includes a single-chain variable fragment (scFv). In some embodiments, the single-chain antigen binding molecule includes a nanobody. It will be appreciated by one skilled in the art that additional single-chain antigen molecules can also be used with any of the methods described herein.

### Antigen-Binding Molecule Display Methods

Various display methods suitable for the methods disclosed herein include, but are not limited to, yeast display, phage display, ribosome display, and mRNA/cDNA display. Generally, there are advantages and disadvantages to each to these various display methods. For example, yeast and phage display are generally more commonly used, and include advantages such as full-length IgGs and post-translation modifications (e.g., glycosylation in yeast); however, library generation in these display methods tend to be more onerous. For example, yeast and phage display methods described herein, require the synthesized DNA libraries to be first cloned into a yeast or phage vector. Subsequently, the yeast or phage is lysed in order to release the cDNA library (e.g., plasmid including the DNA library). In some embodiments, the yeast or phage library is lysed at about the same time as the biological sample (e.g., tissue section) is permeabilized (see, e.g., Fiskin, E. et al., Single-cell multimodal profiling of proteins and chromatin accessibility using PHAGE-ATAC, bioRxiv preprint doi: https://doi.org/10.1101/2020.10.01.322420, (2021)). Additional methods such as ribosome display, discussed below, and mRNA/cDNA display include advantages such as rapidly generating large libraries and tethering translated proteins to their own mRNA.

mRNA/cDNA or mRNA display is a robust *in vitro* display technology that converts an unstable mRNA-protein fusion (mRNA display) to a stable mRNA/cDNA-protein fusion (e.g., cDNA display) where the cDNA is covalently linked to its encoded protein using a puromycin linker (Ueno and Nemoto, cDNA display: rapid stabilization of mRNA display, Methods Mol Biol., 805: 113-35 (2012)). Synthesis of an mRNA display library begins with synthesis of a DNA library. A DNA library for any protein or small peptide of interest (e.g., an antigen-binding molecule) can be synthesized by various methods, including solid-phase synthesis followed by PCR amplification. As an example, DNA libraries encoding sequences for nanobodies and associated display methods are described in Doshi, R., et al., In vitro nanobody discovery for integral membrane protein targets. Sci Rep, 4, 6760 (2014). Generally, members of the DNA library have a transcription site (e.g., a T7 RNA polymerase transcription site, a T3 RNA polymerase transcription site, an SP6 RNA polymerase transcription site) and a ribosomal binding site at their 5' end. The promoter (e.g., a RNA promoter such as T7 RNA promoter, SP6 RNA promoter, T3 RNA promoter) allows large-scale *in vitro* transcription to transcribe the DNA library into an mRNA nucleic acid library with a polymerase (e.g., a T7 RNA polymerase, a T3 RNA polymerase, a SP6 RNA polymerase, etc.). Also, the ribosomal binding site in the 5'-untranslated region (5' UTR) can be designed according to the *in vitro* translation system to be used (e.g., prokaryotic or eukaryotic).

As such, mRNA display is a method for *in vitro* protein/peptide evolution to generate molecules that can bind to a desired target. For example, mRNA display can be used to generate a large library of antigen-binding molecules and screened for potential against healthy (e.g., control) and or diseased (e.g., tumorous) biological samples. The mRNA display method results in translated peptides or proteins that are associated with their mRNA via a puromycin linkage. Different methods of screening can be performed, including testing binding of the conjugate(s) to an immobilized target in a selection step, such as affinity chromatography. The mRNA-protein conjugates that bind can be reverse transcribed to cDNA and their sequence amplified via a polymerase chain reaction, resulting in a nucleotide sequence that encodes a peptide with high affinity for the molecule of interest.

Generally, puromycin is used in mRNA display to inhibit translation that results in an mRNA-protein (e.g., antigen-binding molecule/nucleic acid) conjugate. Puromycin is an analogue of the 3' end of a tyrosyl-transfer RNA (tRNA) where a portion of its structure mimics an adenosine molecule and another portion mimics a tyrosine molecule. As compared to the cleavable ester bond found in tyrosyl-tRNA, puromycin has a non-hydrolysable amide bond, and as a result, puromycin interferes (e.g., halts) translation and the conjugate molecule is released from the ribosome.

The present disclosure features display methods including yeast display, phage display, ribosomal display, and/or mRNA/cDNA display with single-chain antigen binding molecules. In some embodiments, the display construct includes an analyte capture sequence capable of binding a capture domain of a capture probe on a substrate. In embodiments utilizing mRNA/cDNA display, the cDNA of the mRNA/cDNA construct includes an analyte capture sequence capable of binding a capture domain of a capture probe on a substrate. In such embodiments, a biological sample (e.g., a tissue section) can be contacted with an array including a plurality of capture probes, where a capture probe of the plurality of capture probes includes: i) a spatial barcode and ii) a capture domain.

In some embodiments, after translation of the mRNA, the mRNA of the mRNA/protein complex is directly captured (e.g., without cDNA synthesis). Provided herein are methods of generating an antigen-binding molecule/nucleic acid conjugate library, where an antigen-binding molecule/nucleic acid conjugate of the antigen-binding molecule/nucleic acid conjugate library includes an antigen-binding molecule linked to a nucleic acid encoding the antigen-binding molecule, the method including: a) providing a plurality of in vitro transcribed mRNAs, wherein an mRNA of the plurality of in vitro transcribed mRNAs comprises in a 5' to 3' direction: (i) an analyte capture sequence that hybridizes to a capture domain of a capture probe, (ii) a sequence encoding the antigen-binding molecule, (iii) a primer binding sequence, (iv) a universal domain attached to a donor oligonucleotide, wherein the donor oligonucleotide comprises a linker having a nucleotide sequence and a protein translation inhibitor; and b) in vitro translating the plurality of in vitro transcribed mRNAs, thereby generating the antigen-binding molecule/nucleic acid conjugate library antigen-binding molecule mRNA.

The methods described herein allow identification of an antigen-binding molecule that selectively binds to an antigen in a biological sample (e.g., a tissue section) in its *in vivo* spatial context, whereas typical screening methods are performed for known targets and in *in vitro* or lysate samples.

mRNA display also generally includes the use of oligonucleotides or other linkers to provide flexibility and proper length for the puromycin to enter the A site of the ribosome. Generally, the linker between the 3' end of an mRNA and the puromycin has to be flexible and long enough to allow the puromycin to enter the A site upon translation of the last codon. In some embodiments, the linker includes a poly(A) sequence. In some embodiments, the linker includes a unique sequence. In some embodiments, the linker is at least 10 nucleotides, at least 15 nucleotides, at least 20 nucleotides, at least 25 nucleotides, at least 30 nucleotides, or at least 35 nucleotides.

As discussed above mRNA display can include the use of linkers, such as oligonucleotide linkers, conjugated to a protein translation inhibitor molecule, such as puromycin. In some embodiments, the methods described herein include a prior step of attaching the donor oligonucleotide to the universal domain via ligation. Various methods of attaching the donor oligonucleotide to the universal domain are known to a person skilled in the art, including for example, splint ligation, Y-ligation, ultraviolet light (UV) crosslinking, and the transcription-translation couple with association of puromycin linker (TRAP) system as described in Huang, Y., et al., RNA Display Methods for the Discovery of Bioactive Macrocycles, Chem. Rev., 119, 10360-10391 (2019).

In some embodiments, the ligation includes ligation via a splint oligonucleotide. In some embodiments, the splint oligonucleotide includes a sequence substantially complementary to the linker of the donor oligonucleotide, or a portion thereof. In some embodiments, the splint oligonucleotide includes a sequence substantially complementary to the universal domain, or a portion thereof. In some embodiments, the ligation includes ligation with a splint oligonucleotide. In some embodiments, the splint oligonucleotide includes a sequence substantially complementary to the linker of the donor oligonucleotide, or a portion thereof and includes a sequence substantially complementary to the universal domain, or a portion thereof.

In some embodiments, the ligating includes Y-ligation. In some embodiments, the linker of the donor oligonucleotide includes a sequence substantially complementary to the universal domain, or a portion thereof. In some embodiments, the method includes ligating the 5' end of the donor oligonucleotide to the 3' end of the universal domain.

In some embodiments, the ligating includes the use of a ligase. In some embodiments, the ligase includes T4 DNA ligase, T4 RNA ligase, Tth DNA ligase, Taq DNA ligase, Thermococcus sp. DNA ligase, Ampligase^{™}, PBCV-1 DNA Ligase, Splint ligase, and a Chlorella virus DNA Ligase, and combinations thereof.

In some embodiments, ligating includes ultra-violet (UV) light cross-linking. In some embodiments, the linker of the donor oligonucleotide includes a sequence that is substantially complementary to the universal domain, or a portion thereof. In some embodiments, the 5' end of the linker includes a UV light photoreactive moiety. In some embodiments, the UV light photoreactive moiety includes psoralen.

In some embodiments, the linker of the donor oligonucleotide includes a sequence that is substantially complementary to the universal domain, or a portion thereof. In some embodiments, the protein translation inhibitor is non-covalently attached to the mRNA.

In some embodiments, the protein translation inhibitor is a protein translation inhibitor molecule. In some embodiments, the protein translation inhibitor molecule is covalently attached to the mRNA. In some embodiments, the protein translation inhibitor molecule is puromycin.

Additional methods of antigen-binding molecule display are known in the art including ribosomal display (*see e.g.,* He and Taussing, Ribosome display: Cell-free protein display technology, Briefings in Functional Genomics and Proteomics, 204-212 (2002)). Generally, ribosomal display is a cell-free system for the *in vitro* selection of proteins and peptides from nucleic acid libraries. A fundamental feature of ribosomal display includes the principle of coupling individual translated proteins to their corresponding mRNA, through the formation of stable protein-ribosome-mRNA (PRM) complexes (e.g., antigen-binding molecule/nucleic acid conjugates). Coupling the protein (e.g., antigen-binding molecule) to its own mRNA permits the simultaneous isolation of a functional protein (e.g., antigen-binding molecule) through affinity for a ligand, such as an antigen, together with the encoding mRNA. The mRNA can then be converted (e.g., reverse transcribed) and amplified as DNA for further manipulation, including repeated cycles or protein expression.

Ribosomal display can display large antigen-binding molecule/nucleic acid libraries without the restriction of bacterial transformation. Another benefit of ribosomal display is that it can allow for generating toxic, proteolytically sensitive, and unstable proteins, and allows the incorporation of modified amino acids at defined positions. In combination with polymerase chain reaction (PCR)-based methods, mutations can be introduced efficiently into the selected DNA pool in subsequent cycles, leading to continuous DNA diversification and antigen-binding molecule selection (e.g., *in vitro* protein evolution). Both prokaryotic and eukaryotic ribosome display systems have been developed and each has its own distinctive features.

Generally, ribosomal display strategies include either (i) addition of antibiotics such as rifampicin and chloramphenicol (for prokaryotic ribosomes) or cycloheximide (for eukaryotic ribosomes) to halt translation at random times or (ii) deletion of the stop codon, typically recognized by release factors which trigger detachment of the polypeptide (e.g., antigen-binding molecule), to stall the ribosome at the end of the mRNA.

Thus, in some embodiments, the protein translation inhibitor does not include a stop codon. For example, the lack of a stop codon in the mRNA which includes a sequence encoding an antigen-binding molecule allows the antigen-binding molecule to be translated, but does not allow antigen-binding molecule to be released from the ribosome. The resulting complex is an antigen-binding molecule complexed with its own mRNA (e.g., antigen-binding molecule/nucleic acid complex) that can then be assayed (e.g., screened) for binding an antigen.

As described above in the various display methods (e.g., ribosome display, mRNA display) a complementary cDNA can be generated that encodes a protein or peptide (e.g., an antigen binding molecule of interest). Thus, in some embodiments, the methods described herein include generating a cDNA complementary to the mRNA (e.g., the nucleic acid of the antigen-binding molecule/nucleic acid conjugate) to produce a double stranded DNA/RNA duplex. In some embodiments, generating the cDNA includes reverse transcribing the mRNA. In some embodiments, reverse transcription includes the use of a primer that hybridizes to a universal domain of the mRNA.

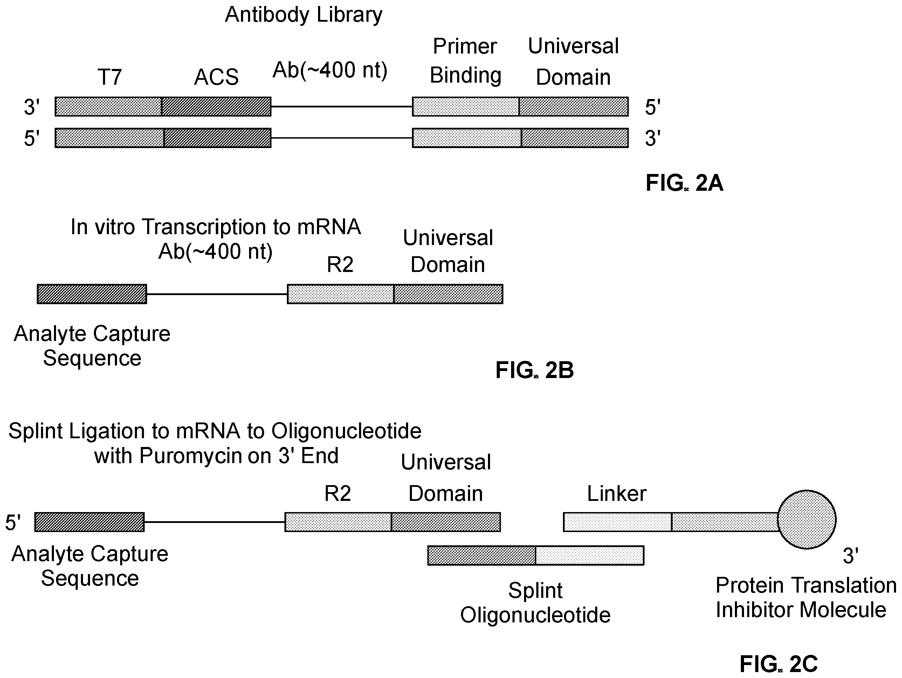

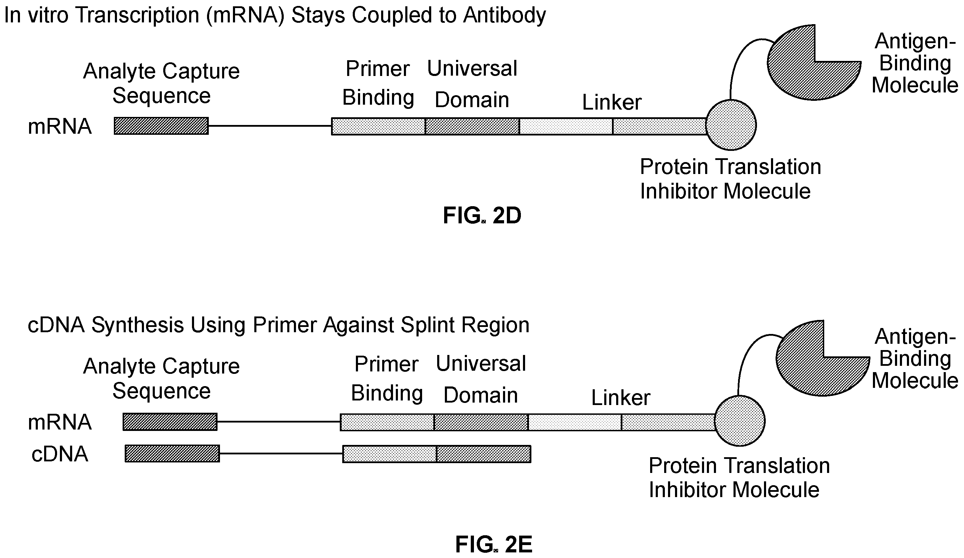

FIGs. 2A-E show an exemplary single-chain antibody nucleic acid library (FIG. 2A) and an exemplary method of generating a display library via *in vitro* transcription of the single-chain antibody nucleic acid library (FIGs. 2B-E). In some embodiments, a member of the nucleic acid library includes in a 5' to 3' direction a primer binding sequence, an analyte capture sequence (ACS) (e.g., an analyte capture sequence that interacts with a capture domain) or a reverse complement thereof, a sequence encoding a single-chain antibody or antigen-binding fragment thereof, a second primer binding site and a universal domain (FIG. 2A). In some embodiments, a member of the nucleic acid library includes an analyte capture sequence (e.g., an analyte capture sequence that interacts with a capture domain) or a reverse complement thereof, a sequence encoding a single-chain antibody or antigen-binding fragment thereof, a primer binding site, and a universal domain. In some embodiments, a member of the nucleic acid library includes a promoter sequence, an analyte capture sequence (e.g., an analyte capture sequence that interacts with a capture domain) or a reverse complement thereof, a sequence encoding a single-chain antibody or antigen-binding fragment thereof, a primer binding site, and a universal domain. The promoter sequence can be a promoter suitable for *in vitro* transcription. Exemplary promoter sequences for *in vitro* transcription include, but are not limited to, an RNA polymerase promoter sequence, e.g., a T7 promoter sequence, SP6 promoter sequence, T3 promoter sequence. While FIG. 2A shows a particular 5' to 3' orientation of the domains, it is appreciated by those skilled in the art that the orientation of the domains can be rearranged in such a manner to capture the mRNA directly after *in vitro* transcription as described above. Additionally, FIG. 2A shows a double-stranded nucleic acid library, however, it is understood by those skilled in the art that the nucleic acid library can be a single-stranded nucleic acid library. Thus, in some embodiments, the nucleic acid library is double-stranded. In some embodiments, the nucleic acid library is single-stranded. In some embodiments, the nucleic acid library (e.g., any of the nucleic acid libraries described herein) are transcribed to mRNA. For example, FIG. 2B shows the resulting mRNA after *in vitro* transcription of a member of the nucleic acid library shown in FIG. 2A. In some embodiments, a protein translation inhibitor molecule and a linker are attached to the 3' end of the transcribed mRNA. FIG. 2C shows an exemplary method of the mRNA of FIG. 2B ligated to a molecule including a linker and a protein translation inhibitor molecule. In some embodiments, the ligation includes a splint oligonucleotide, however, other methods as described herein can also be used. FIG. 2C shows an exemplary method where the ligation is facilitated by a splint oligonucleotide, although other methods of attaching the mRNA and the molecule including the linker and protein translation inhibitor molecule are known in the art. For example, other methods of attaching the mRNA and the molecule including the linker and the protein translation inhibitor molecule including Y-ligation or the TRAP system as described herein. **FIG. 2D** shows the resulting antigen-binding molecule/nucleic acid conjugate, however, other methods and additional orientations of the domains shown in **FIG. 2D** are also contemplated by the present disclosure. **FIG. 2E** shows a complementary cDNA synthesized to the nucleic acid portion of the antigen-binding/nucleic acid conjugate. In some embodiments, a cDNA is synthesized complementary to the mRNA sequence of the antigen-binding/nucleic acid conjugate. In some embodiments, a cDNA is not synthesized to the mRNA sequence of the antigen-binding/nucleic acid conjugate. For example, the mRNA (e.g., the nucleic acid sequence encoding the mRNA) can be designed in such a way and with the domains oriented in such a way as described herein to enable direct capture of the nucleic acid of the antigen-binding/nucleic acid conjugate without the synthesis of cDNA as shown in **FIG. 2E**.

### Screening

The display methods described herein can be further used in various screening assays. For example, antigen-binding molecule/nucleic acid conjugates produced by any of the display methods described herein can be screened against a particular target of interest. Additionally, antigen-binding molecule/nucleic acid conjugates can be screened against diseased biological samples (e.g., tumorous tissue) to identify novel antigen-binding molecules that bind targets expressed and/or overexpressed in diseased cells or tissues that may be valuable in future therapeutic applications. For example, antigen-binding molecules can be first positively screened on healthy biological samples (e.g., control biological samples) to potentially identify any antigen-binding molecules with an affinity for a target present in a healthy biological sample. Further, non-binding antigen-binding molecules can be removed from the healthy biological sample and collected (e.g., negative selection) and assayed for binding to a diseased biological sample (e.g., positive selection on a tumorous biological sample). Additionally, while various display methods are typically performed on lysates for a particular target, methods of spatially capturing antigen-binding molecules (e.g., nucleic acids (e.g., cDNA) encoding antigen-binding molecules) from a biological sample (e.g., any of the biological samples described herein) are still needed. For example, screening is generally performed *in vitro* on selected cell lines and/or lysates and may not reflect the *in vivo* context. However, in contrast, screening performed on a biological sample (e.g., a tissue section) where the antigens, including known and unknown antigens, are embedded within a complex tissue environment can provide *in vivo* context lacking in cell lines and/or lysate samples.

Provided herein are methods of identifying an antigen-binding molecule that selectively binds to an antigen in a biological sample, the method including: a) providing an array including a plurality of capture probes, where a capture probe of the plurality includes (i) a spatial barcode and (ii) a capture domain; b) contacting the biological sample with members of an antigen-binding molecule/nucleic acid conjugate library, where an antigen-binding molecule/nucleic acid conjugate of the library includes an antigen-binding molecule linked to a nucleic acid encoding the antigen-binding molecule, and where the nucleic acid includes a cDNA/RNA duplex, where the RNA strand of the duplex comprises: i) a sequence complementary to the analyte capture sequence which binds a capture domain of a capture probe, ii) a sequence encoding an antigen-binding molecule, iii) a primer binding sequence, and iv) a universal domain attached to a donor oligonucleotide, where the donor oligonucleotide includes a linker having a nucleotide sequence and a protein translation inhibitor, c) removing one or more unbound antigen-binding molecule/nucleic acid conjugates from the biological sample; and d) capturing one or more cDNAs of the antigen-binding molecule/nucleic acid conjugates onto one or more capture probes of the array, where an analyte capture sequence of the cDNA binds to the capture domain of the capture probe.

In some embodiments, the method includes determining (i) the sequence of the spatial barcode or a complement thereof, and (ii) all or a portion of the sequence of the cDNA, or a complement thereof. In some embodiments, the method includes using the determined sequences of (i) the sequence of the spatial barcode or a complement thereof and (ii) all or a portion of the sequence of the cDNA or a complement thereof to identify an antigen-binding molecule that selectively binds to an antigen in the biological sample and determine the location of the antigen in the biological sample. In some embodiments, the determining includes sequencing. In some embodiments, step (c) is performed before (a), after (a), or after (b).

In some embodiments, the members of the antigen-binding molecule/nucleic acid conjugate library were selected by a prior step of contacting a control biological sample with the antigen-binding molecule/nucleic acid conjugate library, and selecting members of the library that did not bind to the control biological sample.

In some embodiments, the biological sample is disposed on the spatial array including the plurality of capture probes. For example, the biological sample (e.g., a tissue section) can be placed directly onto the spatial array, where the spatial array includes the plurality of capture probes. In some embodiments, the biological sample is disposed on a substrate (e.g., a separate substrate from the spatial array). For example, a biological sample can be disposed on a second substrate different from the substrate including the spatial array including the plurality of capture probes. In some embodiments, the method includes aligning the substrate (e.g., the substrate with a biological sample) with the spatial array, such that at least a portion of the biological sample is aligned with at least a portion of the spatial array. In such embodiments, the biological sample and the spatial array are in a "sandwich" configuration where either the spatial array or the biological sample are on the bottom or top position of the sandwich configuration.

In some embodiments, the control biological sample is a healthy biological sample. In some embodiments, the biological sample includes one or more healthy regions and one or more disease regions.

In some embodiments, biological sample is a diseased biological sample. In some embodiments, the diseased biological sample is a tumorous biological sample.

In some embodiments, the method includes releasing the cDNA from the antigen-binding molecule/nucleic acid conjugate prior to the capturing step. In some embodiments, the releasing includes an RNase. In some embodiments, the RNase is selected from the group consisting of RNase A, RNase C, RNase H, and RNase I.

In some embodiments, the releasing includes heat.

In some embodiments, the method includes permeabilizing the biological sample, the test biological sample (e.g., a diseased biological sample, a tumorous biological sample), or both.

In some embodiments, the method includes imaging the biological sample. In some embodiments, the method includes staining the biological sample (e.g., staining by any of the methods described herein). In some embodiments, the staining includes hematoxylin and eosin (H&E) staining.

In some embodiments, the array includes one or more features. In some embodiments, the one or more features includes a bead. In some embodiments, the capture probe includes any one or more of: a cleavage domain, a unique molecular identifier (UMI), one or more functional domain, and combinations thereof.

In some embodiments, the antigen-binding molecule is a single-chain antigen-binding molecule. In some embodiments, the single-chain antigen-binding molecule includes a single-chain variable fragment (scFv). In some embodiments, the single-chain antigen-binding molecule includes a nanobody.

In some embodiments, the cDNA complementary to the nucleic acid of the antigen-binding molecule/nucleic acid conjugate is generated by reverse transcription.

The methods described herein can also be multiplexed, that is, capture of additional analytes or analyte derivatives in addition to capturing nucleic acid (e.g., cDNA) encoding an antigen-binding molecule on a spatial array. Thus, for example, additional nucleic acid present in the biological sample can also be captured on a spatial array and/or a nucleic acid derivative, such as for example, RNA templated ligation (RTL) probes that hybridize to a target nucleic acid and where one probe includes an analyte capture sequence that binds to a capture domain of a capture probe. Similarly, protein capture via an analyte capture agent can also be multiplexed with the methods described herein.

In some embodiments, the method includes capturing a nucleic acid analyte or derivative thereof from the biological sample, where the nucleic acid analyte binds to the capture domain of the capture probe.

In some embodiments, the nucleic acid analyte, or derivative thereof, includes DNA. In some embodiments, the nucleic acid analyte, or derivative thereof, includes RNA.

In some embodiments, the method includes (a) contacting a first probe and a second probe with the biological sample, wherein the first probe and the second probe each include one or more sequences that are substantially complementary to sequences of an analyte, and wherein the second probe includes a capture probe capture domain that is complementary to all or a portion of the capture domain; (b) hybridizing the first probe and the second probe to the analyte; (c) generating a ligation product by ligating the first probe and the second probe; (d) releasing the ligation product from the analyte; (e) hybridizing the ligation product to the capture domain; and (g) determining (i) all or part of the sequence of the ligation product bound to the capture domain, or a complement thereof, and (ii) all of the sequence of the spatial barcode, or a complement thereof, and using the determined sequences of (i) and (ii) to identify the location of the analyte in the biological sample.

In some embodiments, the methods include identifying the antigen bound by the antigen-binding molecule/nucleic acid complex. In some embodiments, the identifying includes mass spectrometry. In some embodiments, the identifying includes determining differential gene expression patterns between the control biological sample and the test biological sample. In some embodiments, the identifying includes determining differential gene expression patterns within the biological sample. For example, in instances where a biological sample includes healthy and diseased regions, differential gene expression patterns within those regions can be determined.

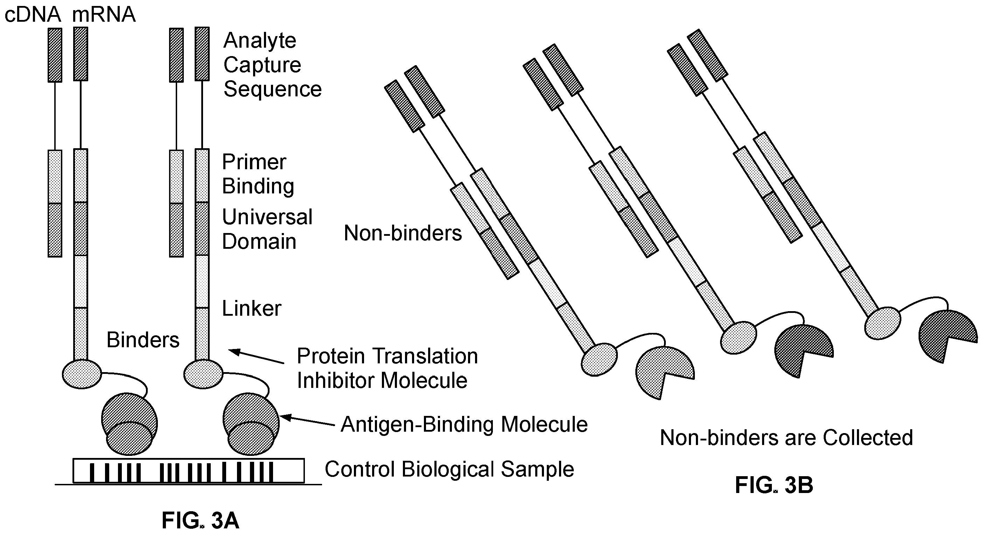

Antigen-binding molecule/nucleic acid conjugates generated as described herein can be screened against a control biological sample (e.g., a healthy biological sample, a non-diseased biological sample). In some embodiments, non-binding antigen-binding molecule/nucleic acid conjugates are removed from the control biological sample and collected for screening against a diseased (e.g., tumor) biological sample. **FIGs. 3A-B**show exemplary antigen-binding molecule/nucleic acid conjugate members generated, as described herein, binding to an antigen in a control biological sample (e.g., a healthy biological sample) (**FIG. 3A**). In some embodiments, the antigen-binding molecule/nucleic acid conjugate members bind to an antigen in a biological sample. In some embodiments, the antigen-binding molecule/nucleic acid conjugate members do not bind to an antigen in a biological sample (e.g., non-binding antigen-binding molecule/nucleic acid conjugates). While **FIG. 3A** shows an exemplary antigen-binding molecule/nucleic acid conjugate binding an antigen in a biological sample it is contemplated by the present disclosure that the antigen-binding molecule/nucleic acid conjugate, in some embodiments, does not include the cDNA as shown. **In** some embodiments, non-binding antigen-binding/nucleic acid conjugates are removed (e.g., washed) from the biological sample. In some embodiments, the non-binding antigen-binding/nucleic acid conjugates are collected (**FIG. 3B**).

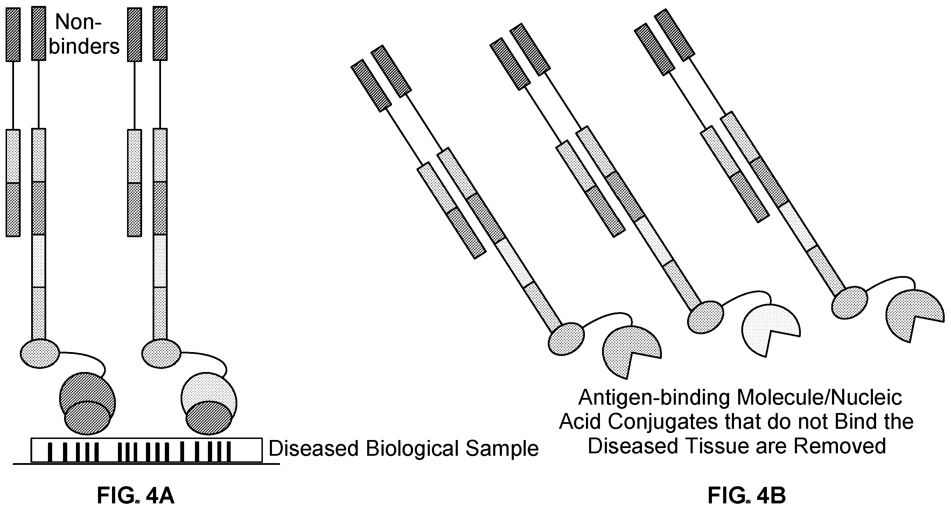

The non-binding antigen-binding molecule/nucleic acid conjugates collected in **FIG. 3B** can be screened against diseased biological samples. Thus, **FIG. 4A** shows such collected non-binding antigen-binding molecule/nucleic acid conjugates screened against a diseased biological sample. In some embodiments, one or more of the collected non-binding antigen-binding molecule/nucleic acid conjugates bind the diseased biological sample. In some embodiments, one or more of the collected non-binding antigen-binding molecule/nucleic acid conjugates do not bind the diseased biological sample. In some embodiments, the antigen-binding molecule/nucleic acid conjugates that do not bind a diseased biological sample are removed (e.g., washed off) from the diseased biological sample as shown in **FIG. 4B**.

In some embodiments, the synthesized cDNA, or a portion thereof, hybridizes to the binding antigen-binding molecule/nucleic acid conjugate members shown in **FIG. 4A** can be released (e.g., released with heat, treated with an RNase (e.g., any suitable RNase)) from the antigen-binding molecule/nucleic acid conjugate. In some embodiments, the released cDNA interacts with (e.g., hybridizes) the capture domain of a capture probe on array including a plurality of capture probes, where a capture probe includes a spatial barcode and a capture domain. For example, the cDNA includes an analyte capture sequence that binds to the capture domain. In some embodiments, no cDNA is synthesized (not shown). In such embodiments, the mRNA of the antigen-binding molecule/nucleic acid conjugate is directly captured (e.g., captured without the synthesis of the cDNA) by the capture probe on the array. In such embodiments, the orientation of the domains shown in **FIG. 2A** can be rearranged (e.g., rearranged as described herein) to facilitate direct capture of the mRNA of the antigen-binding molecule/nucleic acid conjugate.

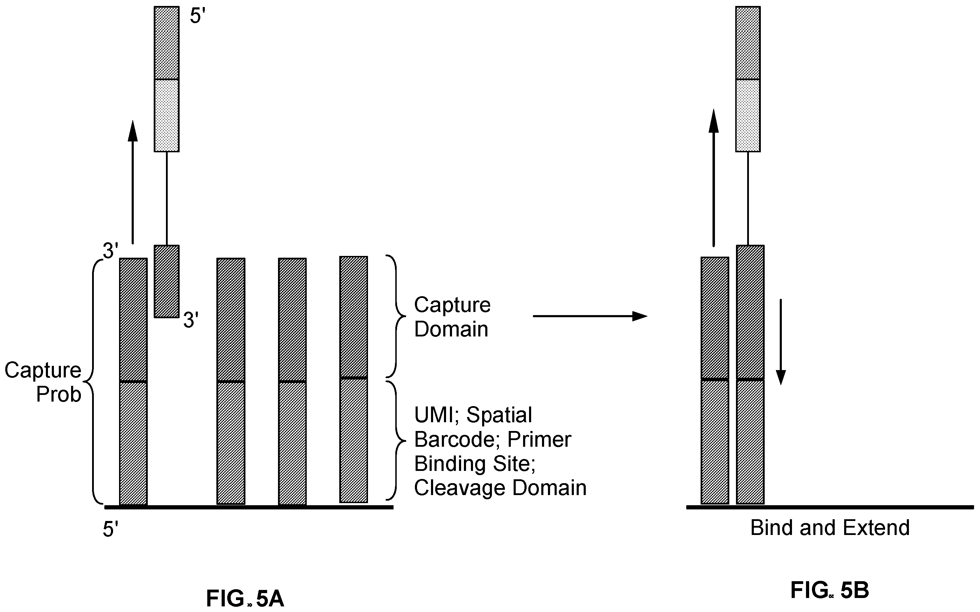

In some embodiments, a capture probe (e.g., a capture probe including a spatial barcode) and a capture domain can bind (e.g., hybridize) with the released cDNA, or portion thereof, as shown in **FIG. 5A**. In some embodiments, the cDNA is the cDNA released (e.g., released by any of the methods described herein) from the antigen-binding molecule/nucleic acid conjugate members. In some embodiments, the cDNA is released via heat. In some embodiments, the cDNA is released via denaturation. In some embodiments, the cDNA is released via RNase digestion (e.g., digested by any of the RNases described herein). In some embodiments, the captured cDNA is extended using the capture probe as a template as shown in **FIG. 5B**. In some embodiments, the capture probe is extended using the captured cDNA as an extension template. In some embodiments, both the capture probe and the cDNA are extended using the cDNA and the capture probe, respectively, as an extension template.

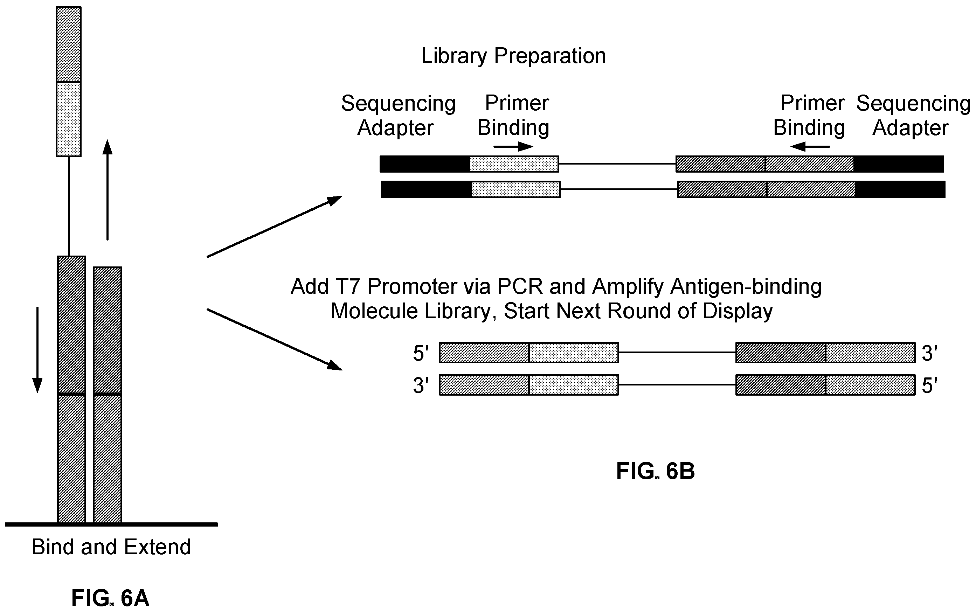

**FIG. 6A** shows extension of the 3' end of the capture probe and the 3' end of the cDNA (**FIG. 6A**) from the antigen-binding molecule/nucleic acid conjugate. **FIG. 6B** shows the resulting nucleic acid library and potential downstream applications including amplifying the nucleic acid library and/or adding a promoter sequence (e.g., RNA polymerase promoter sequence, e.g., a T7, SP6 or T3 promoter sequence) to the nucleic acid library (or amplified nucleic acid library) for another round of antigen-binding molecule screening.

In some embodiments, the resulting nucleic acid library is amplified. In some embodiments, all or a portion of the resulting nucleic acid library (or amplified nucleic acid library) is sequenced according to known methods in the art to identify the antigen-binding molecule. In some embodiments, the resulting nucleic acid library (or amplified nucleic acid library) has a primer binding sequence added and is amplified for another round of antigen-binding molecule screening. In some embodiments, the resulting nucleic acid library (or amplified nucleic acid library) has a promoter sequence (e.g., RNA polymerase promoter sequence, e.g., a T7, SP6, or T3 promoter sequence) added and is amplified for another round of antigen-binding molecule screening.

### Systems

As described herein, spatial analysis can be performed using dedicated hardware and/or software, such as any of the systems described in Sections (II)(e)(ii) and/or (V) of WO 2020/176788 and/or U.S. Patent Application Publication No. 2020/0277663, or any of one or more of the devices or methods described in Sections *Control Slide for Imaging, Methods of Using Control Slides and Substrates for, Systems of Using Control Slides and Substrates for Imaging,* and/or *Sample and Array Alignment Devices and Methods, Informational labels* of WO 2020/123320.

Suitable systems for performing spatial analysis can include components such as a chamber (e.g., a flow cell or sealable, fluid-tight chamber) for containing a biological sample. The biological sample can be mounted for example, in a biological sample holder. One or more fluid chambers can be connected to the chamber and/or the sample holder via fluid conduits, and fluids can be delivered into the chamber and/or sample holder via fluidic pumps, vacuum sources, or other devices coupled to the fluid conduits that create a pressure gradient to drive fluid flow. One or more valves can also be connected to fluid conduits to regulate the flow of reagents from reservoirs to the chamber and/or sample holder.

Thus, provided herein are systems including a) an antigen-binding molecule/nucleic acid conjugate produced by any of the methods described herein; and b) an array including a plurality of capture probes, wherein a capture probe of the plurality includes a capture domain that binds specifically (e.g., hybridizes) to the analyte capture domain and a spatial barcode. In some embodiments, the system includes reverse transcriptase.

In some embodiments, the system includes ribonucleotide triphosphates, deoxyribonucleotide triphosphates, and one or more buffers.

In some embodiments, the system includes one or more permeabilization reagents.

In some embodiments, the one or more permeabilization reagents includes an RNase, a DNase, a lipase, a protease, and combinations thereof.

In some embodiments, the capture probe includes one or more functional domains, a cleavage domain, a unique molecular identifier, and combinations thereof.

### Compositions

The present disclosure also features compositions including antibody and/or antigen-binding molecule nucleic acid libraries and antigen-binding/nucleic acid conjugates generated therefrom.

Thus, provided herein are compositions including an in vitro transcribed mRNA in a 5' to 3' direction: (i) a sequence complementary to an analyte capture sequence that hybridized to a capture domain of a capture probe, (ii) a sequence encoding an antigen-binding molecule, (iii) a primer binding sequence, and (iv) a universal sequence. In some embodiments, the nucleic acid library is single-stranded. In some embodiments, the nucleic acid library is double-stranded.

In some embodiments, the composition includes a donor oligonucleotide attached to the universal sequence of the mRNA. In some embodiments, the donor oligonucleotide includes a linker having a sequence and a protein translation inhibitor. In some embodiments, the protein translation inhibitor includes a translation inhibitor molecule. In some embodiments, the translation inhibitor molecule includes puromycin. In some embodiments, the protein translation inhibitor does not include a stop codon. For example, the lack of a stop codon tethers the translated protein to its own mRNA non-covalently.

In some embodiments, the donor oligonucleotide is attached the universal sequence of the mRNA with a splint oligonucleotide. In some embodiments, the splint oligonucleotide includes a substantially complementary sequence to the universal domain and a substantially complementary sequence to the linker of the donor oligonucleotide.

In some embodiments, the nucleic acid library is transcribed (e.g., transcribed into mRNA). In some embodiments, a protein translation inhibitor molecule is attached to the transcribed mRNA. In some embodiments, a linker is disposed between the mRNA and the protein translation inhibitor molecule.

Also provided herein are compositions including an antigen-binding molecule/nucleic acid conjugate including (i) a sequence complementary to an analyte capture sequence, (ii) a sequence encoding an antigen-binding molecule, (iii) a primer binding sequence, (iv) a constant sequence; (v) a protein translation inhibitor molecule; and (vi) the antigen-binding molecule.

In some embodiments, the antigen-binding molecule includes a single-chain antigen binding molecule. In some embodiments, the single-chain antigen binding molecule includes an scFv. In some embodiments, the single-chain antigen binding molecule includes a nanobody.

In some embodiments, the composition further includes a cDNA hybridized to the nucleic acid of the antigen-binding molecule/nucleic acid conjugate. In some embodiments, the cDNA is generated by reverse transcription. For example, a complementary cDNA can be generated by reverse transcription to the nucleic acid portion of the antigen-binding molecule/nucleic acid conjugate.

Also provided herein are compositions including a) an array including a plurality of capture probes, where a capture probe includes: (i) a spatial barcode; and (ii) a capture domain; and b) a cDNA including in the 5' to 3' direction: (i) a sequence complementary to a universal domain; (ii) a sequence complementary to a primer binding site; (iii) a sequence complementary to a sequence encoding an antigen-binding molecule; and (iv) an analyte capture sequence, wherein the analyte capture sequence is bound to the capture domain of the capture probe.

Also provided herein are compositions including: a) an array including a plurality of capture probes, wherein a capture probe includes: (i) a spatial barcode; and (ii) a capture domain; and b) an extended cDNA product including in the 5' to 3' direction: (i) a sequence complementary to a universal domain; (ii) a sequence complementary to a primer binding site;
(iii) a sequence complementary to a sequence encoding an antigen-binding molecule; (iv) an analyte capture sequence, where the analyte capture sequence is bound to the capture domain of the capture probe; (v) a sequence complementary the capture probe; and c) an extended capture probe including a sequence complementary to the cDNA using the cDNA as an extension template.

In some embodiments, the capture probe (e.g., any of the capture probes described herein) includes one or more functional domains, a unique molecular identifier, a cleavage domain, and combinations thereof.

### Kits

In addition to the methods, systems, and compositions disclosed herein, the present disclosure also describes kits of the methods, systems, and compositions described herein. Thus, provided herein are kits including (i) a spatial array including a plurality of capture probes, wherein a capture probe of the plurality of capture probes includes a spatial barcode and a capture domain; (ii) a plurality of splint oligonucleotides; and (iii) a plurality of donor oligonucleotides, wherein a donor oligonucleotide of the plurality of donor oligonucleotides includes a universal domain, a linker, and a protein translation inhibitor.

In some embodiments, the kit includes a ligase (e.g., any of the ligases described herein).

In some embodiments, the kit includes one or more permeabilization reagents. In some embodiments, the one or more permeabilization reagents comprises an RNase, a DNase, a protease, a lipase, and combinations thereof.

In some embodiments, the kit includes one or more DNA polymerases. In some embodiments, the kit includes one or more reverse transcriptases. In some embodiments, the kit includes a template switch oligonucleotide.

In some embodiments, the protein translation inhibitor is puromycin.

In some embodiments, the kit includes a primer that binds to the universal domain.

### EXAMPLES

### Example 1. Generation of Antigen-Binding/Nucleic Acid Conjugate Library

**FIGs. 2A-E**show an exemplary single-chain antibody nucleic acid library (**FIG. 2A**) and an exemplary method of generating a display library via *in vitro* transcription of the single-chain antibody nucleic acid library (**FIG. 2B-E**). **FIG. 2A** shows a nucleic acid library where a member of the nucleic acid library includes in a 5' to 3' direction a primer binding sequence, an analyte capture sequence (e.g., an analyte capture sequence that binds to a capture domain) or a complement thereof, a sequence encoding a single-chain antibody or antigen-binding fragment, a second primer binding site and a universal domain. While **FIG. 2A** shows a double-stranded nucleic acid library, it is understood by those skilled in the art that the nucleic acid library can be a single-stranded nucleic acid library. **FIG. 2B** shows the resulting mRNA after *in vitro* transcription of a member of the nucleic acid library. **FIG. 2C** shows the mRNA of **FIG. 2B** ligated to a molecule including a linker and a protein translation inhibitor molecule. **FIG. 2C** shows the ligation facilitated by a splint oligonucleotide, although other methods of attaching the mRNA and the molecule including the linker and protein translation inhibitor molecule are known in the art, including Y-ligation and the TRAP system (as described herein), and the resulting antigen-binding molecule/nucleic acid conjugate (**FIG. 2D**). **FIG. 2E** shows a complementary cDNA synthesized to the nucleic acid portion of the antigen-binding/nucleic acid conjugate.

### Example 2. Spatial Antibody or Antigen-Binding Fragment Screening

Antigen-binding molecule/nucleic acid conjugates generated in Example 1 are screened against healthy biological samples (e.g., a control biological sample) and non-binding antigen-binding molecule/nucleic acid conjugates are removed and collected for screening against diseased (e.g., tumor) biological samples. For example, **FIGs. 3A-B**show exemplary antigen-binding molecule/nucleic acid conjugate members generated in Example 1 binding to an antigen in a biological sample (**FIG. 3A**). **FIG. 3A** shows binding to a control biological sample (e.g., a healthy biological sample) and removal and collection of non-binding antigen-binding molecule/nucleic acid conjugate members (**FIG. 3B**).

As shown in **FIGs. 4A-B** non-binding antigen-binding molecule/nucleic acid conjugate members collected in **FIG. 3B** are assessed for binding to a diseased biological sample (**FIG. 4A**). Non-binding antigen-binding molecule/nucleic acid conjugate members are removed from the biological sample (e.g., washed off) (**FIG. 4B**).

The synthesized cDNA, or a portion thereof, hybridized to the binding antigen-binding molecule/nucleic acid conjugate members shown in **FIG. 4A** is released (e.g., released with heat, treated with an RNase (e.g., any RNase described herein)) from the antigen-binding molecule/nucleic acid conjugate. The released cDNA binds with (e.g., hybridizes) to the capture domain of a capture probe on array including a plurality of capture probes, where a capture probe includes a spatial barcode and a capture domain. For example, the cDNA includes an analyte capture sequence that binds to the capture domain.

**FIGs. 5A-B**show a capture probe (e.g., the capture domain of a capture probe) hybridized to the analyte capture sequence of the cDNA from the antigen-binding molecule/nucleic acid conjugate members (**FIG. 5A**). **FIG. 5B** shows extension of the 3' end of the capture probe and the 3' end of the cDNA.

**FIGs. 6A-B** show extension of the 3' end of the capture probe and the 3' end of the cDNA (**FIG. 6A**) and the resulting nucleic acid library in **FIG. 6B**. In some examples, the resulting nucleic acid library is sequenced according to known methods in the art to identify the antigen-binding molecule. In some examples, the resulting nucleic acid library has a primer binding sequence added and is amplified for another round of antigen-binding molecule screening as described herein.

## Claims

1. A method of identifying an antigen-binding molecule that selectively binds to an antigen in a tissue sample, the method comprising:
a) providing an array comprising a plurality of capture probes, wherein a capture probe of the plurality comprises (i) a spatial barcode and (ii) a capture domain;
b) contacting the tissue sample with members of an antigen-binding molecule/nucleic acid conjugate library, wherein an antigen-binding molecule/nucleic acid conjugate of the library comprises an antigen binding molecule linked to a nucleic acid encoding the antigen-binding molecule, and wherein the nucleic acid comprises a cDNA/RNA duplex, wherein the RNA strand of the duplex comprises:
i) a sequence complementary to an analyte capture sequence which binds the capture domain of the capture probe,
ii) a sequence encoding the antigen-binding molecule,
iii) a primer binding sequence, and
iv) a universal domain attached to a donor oligonucleotide, wherein the donor oligonucleotide comprises a linker having a nucleotide sequence and a protein translation inhibitor,
wherein the members of the antigen-binding molecule/nucleic acid conjugate library were selected by a prior step of contacting a control tissue sample with the antigen-binding molecule/nucleic acid conjugate library, and selecting members of the library that did not bind to the control tissue sample,
c) removing one or more unbound antigen-binding molecule/nucleic acid conjugates from the tissue sample;
d) capturing one or more cDNAs of the antigen-binding molecule/nucleic acid conjugates onto one or more capture probes of the array, wherein the analyte capture sequence of the cDNA binds to the capture domain of the capture probe; and
e) determining (i) the sequence of the spatial barcode or a complement thereof, and (ii) all or a portion of the sequence of the cDNA, or a complement thereof, and using the determined sequences of (i) and (ii) to identify the antigen-binding molecule that selectively binds to the antigen in the tissue sample.

2. The method of claim 1, wherein the determining comprises sequencing.

3. The method of claim 1 or 2, wherein step (c) is performed before (a), after (a), or after (b).

4. The method of any one of claims 1-3, wherein the control tissue sample is a healthy tissue sample.

5. The method of any one of claims 1-4, wherein the tissue sample comprises one or more healthy regions and one or more disease regions, or wherein the tissue sample is a diseased tissue sample, optionally wherein the diseased tissue sample is a tumorous tissue sample.

6. The method of any one of claims 1-5, wherein the method further comprises releasing the cDNA from the antigen-binding molecule/nucleic acid conjugate prior to the capturing step, optionally wherein the releasing comprises;
(i) use of an RNase, optionally wherein the RNase is selected from the group consisting of RNase A, RNase C, RNase H, or RNase I; or
(ii) heat.

7. The method of any one of claims 1-6, wherein the method further comprises:
(i) permeabilizing the tissue sample;
(ii) imaging the tissue sample; and/or
(iii) staining the tissue sample, optionally wherein the staining comprises hematoxylin and eosin (H&E) staining.

8. The method of any one of claims 1-7, wherein the array comprises one or more features, optionally wherein the one or more features comprises a bead.

9. The method of any one of claims 1-8, wherein the capture probe comprises any one or more of: a cleavage domain, a unique molecular identifier (UMI), one or more functional domains, and combinations thereof.

10. The method of any one of claims 1-9, wherein the antigen-binding molecule is a single-chain antigen-binding molecule, optionally wherein the single-chain antigen-binding molecule comprises a single-chain variable fragment (scFv) or a nanobody.

11. The method of any one of claims 1-10, wherein the cDNA complementary to the nucleic acid of the antigen-binding molecule/nucleic acid conjugate is generated by reverse transcription.

12. The method of any one of claims 1-11, wherein the method further comprises capturing a nucleic acid analyte or derivative thereof from the tissue sample, wherein the nucleic acid analyte binds to the capture domain of the capture probe, optionally wherein the nucleic acid analyte, or derivative thereof, comprises DNA or RNA.

13. The method of any one of claims 1-12, wherein the method further comprises:
(a) contacting a first probe and a second probe with the tissue sample, wherein the first probe and the second probe each comprise one or more sequences that are substantially complementary to sequences of an analyte, and wherein the second probe comprises a capture probe capture domain that is complementary to all or a portion of the capture domain;
(b) hybridizing the first probe and the second probe to the analyte;
(c) generating a ligation product by ligating the first probe and the second probe;
(d) releasing the ligation product from the analyte;
(e) hybridizing the ligation product to the capture domain; and
(g) determining (i) all or part of the sequence of the ligation product bound to the capture domain, or a complement thereof, and (ii) the sequence of the spatial barcode, or a complement thereof, and using the determined sequences of (i) and (ii) to identify the location of the analyte in the tissue sample.

14. The method of any one of claims 1-13, wherein the method further comprises identifying the antigen bound by the antigen-binding molecule/nucleic acid conjugate, optionally wherein the identifying comprises mass spectrometry, and/or wherein the identifying comprises determining differential gene expression patterns between the control tissue sample and the diseased tissue sample or within the tissue sample.

15. The method of any one of claims 1-14, wherein the tissue sample is disposed on the spatial array comprising the plurality of capture probes., or wherein the tissue sample is disposed on a substrate optionally further comprising aligning the substrate with the spatial array, such that at least a portion of the tissue sample is aligned with at least a portion of the spatial array.

## Patentansprüche

1. Verfahren des Identifizierens eines antigenbindenden Moleküls, das selektiv an ein Antigen in einer Gewebeprobe bindet, das Verfahren umfassend:
a) Bereitstellen einer Anordnung umfassend eine Mehrzahl von Erfassungssonden, wobei eine Erfassungssonde der Mehrzahl (i) einen räumlichen Barcode und (ii) eine Erfassungsdomäne umfasst;
b) Inkontaktbringen der Gewebeprobe mit Mitgliedern einer antigenbindendes Molekül/Nukleinsäure-Konjugat-Bibliothek, wobei ein antigenbindendes Molekül/Nukleinsäure-Konjugat der Bibliothek ein antigenbindendes Molekül umfasst, das mit einer Nukleinsäure verknüpft ist, die das antigenbindende Molekül kodiert, und wobei die Nukleinsäure einen cDNA/RNA-Duplex umfasst, wobei der RNA-Strang des Duplexes umfasst:
i) eine Sequenz komplementär zu einer Analyt-Erfassungssequenz, die die Erfassungsdomäne der Erfassungssonde bindet,
ii) eine Sequenz, die das antigenbindende Molekül kodiert,
iii) eine Primerbindungssequenz, und
iv) eine universelle Domäne, angelagert an ein Donor-Oligonukleotid, wobei das Donor-Oligonukleotid einen Linker umfasst, der eine Nukleotidsequenz und einen Protein-Translationsinhibitor aufweist,
wobei die Mitglieder der antigenbindendes Molekül/Nukleinsäure-Konjugat-Bibliothek durch einen vorherigen Schritt des Kontaktierens einer Kontrollgewebeprobe mit der antigenbindendes Molekül/Nukleinsäure-Konjugat-Bibliothek und des Auswählens von Mitgliedern der Bibliothek, die nicht an die Kontrollgewebeprobe gebunden haben, ausgewählt wurden,
c) Entfernen eines oder mehrerer ungebundener antigenbindender Moleküle/Nukleinsäure-Konjugate aus der Gewebeprobe;
d) Erfassen einer oder mehrerer cDNAs der antigenbindendes Molekül/Nukleinsäure-Konjugate auf einer oder mehreren Erfassungssonden der Anordnung, wobei die Analyt-Erfassungssequenz der cDNA an die Erfassungsdomäne der Erfassungssonde bindet; und
e) Bestimmen (i) der Sequenz des räumlichen Barcodes oder eines Komplements davon und (ii) der gesamten oder eines Abschnitts der Sequenz der cDNA oder eines Komplements davon und Verwenden der bestimmten Sequenzen von (i) und (ii) für ein Identifizieren des antigenbindenden Moleküls, das selektiv an das Antigen in der Gewebeprobe bindet.

2. Verfahren nach Anspruch 1, wobei das Bestimmen Sequenzieren umfasst.

3. Verfahren nach Anspruch 1 oder 2, wobei Schritt (c) vor (a), nach (a) oder nach (b) durchgeführt wird.

4. Verfahren nach einem der Ansprüche 1 bis 3, wobei die Kontrollgewebeprobe eine gesunde Gewebeprobe ist.

5. Verfahren nach einem der Ansprüche 1 bis 4, wobei die Gewebeprobe eine oder mehrere gesunde Regionen und eine oder mehrere Erkrankungsregionen umfasst, oder wobei die Gewebeprobe eine erkrankte Gewebeprobe ist, optional wobei die erkrankte Gewebeprobe eine tumoröse Gewebeprobe ist.

6. Verfahren nach einem der Ansprüche 1 bis 5, wobei das Verfahren ferner ein Freisetzen der cDNA von dem antigenbindendes Molekül/Nukleinsäure-Konjugat vor dem Erfassungsschritt umfasst, wobei das Freisetzen optional umfasst;
(i) Verwenden einer RNase, wobei die RNase optional ausgewählt ist aus der Gruppe bestehend aus RNase A, RNase C, RNase H oder RNase I; oder
(ii) Wärme.

7. Verfahren nach einem der Ansprüche 1 bis 6, wobei das Verfahren ferner umfasst:
(i) Permeabilisieren der Gewebeprobe;
(ii) Bildgebung der Gewebeprobe; und/oder
(iii) Anfärben der Gewebeprobe, wobei die Anfärbung optional eine Hämatoxylin- und Eosin(H&E)-Färbung (H&E) umfasst.

8. Verfahren nach einem der Ansprüche 1 bis 7, wobei die Anordnung ein oder mehrere Merkmale umfasst, wobei das eine oder die mehreren Merkmale optional ein Kügelchen umfassen.

9. Verfahren nach einem der Ansprüche 1 bis 8, wobei die Erfassungssonde eines oder mehrere umfasst von: einer Spaltungsdomäne, einer eindeutigen molekularen Kennung (UMI), einer oder mehrerer funktioneller Domänen und Kombinationen davon.

10. Verfahren nach einem der Ansprüche 1 bis 9, wobei das antigenbindende Molekül ein einzelkettiges antigenbindendes Molekül ist, wobei das einzelkettige antigenbindende Molekül optional ein einzelkettiges variables Fragment (scFv) oder einen Nanokörper umfasst.

11. Verfahren nach einem der Ansprüche 1 bis 10, wobei die zu der Nukleinsäure des antigenbindenden Moleküls/Nukleinsäure-Konjugats komplementäre cDNA durch reverse Transkription erzeugt wird.

12. Verfahren nach einem der Ansprüche 1 bis 11, wobei das Verfahren ferner Erfassen eines Nukleinsäureanalyts oder eines Derivats davon aus der Gewebeprobe umfasst, wobei der Nukleinsäureanalyt an die Erfassungsdomäne der Erfassungssonde bindet, wobei der Nukleinsäureanalyt oder das Derivat davon optional DNA oder RNA umfasst.

13. Verfahren nach einem der Ansprüche 1 bis 12, wobei das Verfahren ferner umfasst:
(a) Inkontaktbringen einer ersten Sonde und einer zweiten Sonde mit der Gewebeprobe, wobei die erste Sonde und die zweite Sonde jeweils eine oder mehrere Sequenzen umfassen, die im Wesentlichen komplementär zu Sequenzen eines Analyten sind, und wobei die zweite Sonde eine Erfassungssonden-Einfangdomäne umfasst, die komplementär zu der gesamten oder einem Abschnitt der Erfassungsdomäne ist;
(b) Hybridisieren der ersten Sonde und der zweiten Sonde an den Analyten;
(c) Erzeugen eines Ligationsprodukts durch Ligieren der ersten Sonde und der zweiten Sonde;
(d) Freisetzen des Ligationsprodukts aus dem Analyten;
(e) Hybridisieren des Ligationsprodukts an die Erfassungsdomäne; und
(g) Bestimmen (i) der gesamten oder eines Abschnitts der Sequenz des an die Erfassungsdomäne gebundenen Ligationsprodukts oder eines Komplements davon und (ii) der Sequenz des räumlichen Barcodes oder eines Komplements davon und Verwenden der bestimmten Sequenzen von (i) und (ii) für ein Identifizieren der Position des Analyten in der Gewebeprobe.

14. Verfahren nach einem der Ansprüche 1 bis 13, wobei das Verfahren ferner Identifizieren des durch das antigenbindende Molekül/Nukleinsäure-Konjugat gebundenen Antigens umfasst, optional wobei das Identifizieren Massenspektrometrie umfasst, und/oder wobei das Identifizieren Bestimmen unterschiedlicher Genexpressionsmuster zwischen der Kontrollgewebeprobe und der erkrankten Gewebeprobe oder innerhalb der Gewebeprobe umfasst.

15. Verfahren nach einem der Ansprüche 1 bis 14, wobei die Gewebeprobe auf der räumlichen Anordnung, umfassend die Mehrzahl von Erfassungssonden, angeordnet ist, oder wobei die Gewebeprobe auf einem Substrat angeordnet ist, optional ferner umfassend Ausrichten des Substrats mit der räumlichen Anordnung, sodass mindestens ein Abschnitt der Gewebeprobe mit mindestens einem Abschnitt der räumlichen Anordnung ausgerichtet ist.

## Revendications

1. Procédé d'identification d'une molécule de liaison à l'antigène qui se lie sélectivement à un antigène dans un échantillon de tissu, le procédé comprenant :
a) la fourniture d'un réseau comprenant une pluralité de sondes de capture, dans lequel une sonde de capture de la pluralité comprend : (i) un code à barres spatial et (ii) un domaine de capture ;
b) la mise en contact l'échantillon de tissu avec des membres parmi d'une banque de conjugués molécule de liaison à l'antigène/acide nucléique, dans lequel un conjugué molécule de liaison à l'antigène/acide nucléique de la banque comprend une molécule de liaison à l'antigène liée à un acide nucléique codant pour la molécule de liaison à l'antigène, et dans lequel l'acide nucléique comprend un duplex ADNc/ARN, dans lequel le brin ARN du duplex comprend :
i) une séquence complémentaire d'une séquence de capture d'analyte qui se lie au domaine de capture de la sonde de capture,
ii) une séquence codant pour la molécule de liaison à l'antigène,
iii) une séquence de liaison d'amorce, et
iv) un domaine universel fixé à un oligonucléotide donneur, dans lequel l'oligonucléotide donneur comprend un lieur ayant une séquence nucléotidique et un inhibiteur de traduction de protéine,
dans lequel les membres de la banque de conjugués molécule de liaison à l'antigène/acide nucléique ont été sélectionnés par une étape préalable de mise en contact d'un échantillon de tissu témoin avec la banque de conjugués molécule de liaison à l'antigène/acide nucléique, et de sélection de membres de la banque qui ne se liaient pas à l'échantillon de tissu témoin,
c) l'élimination d'un ou plusieurs conjugués molécule de liaison à l'antigène/acide nucléique non liés de l'échantillon de tissu ;
d) la capture d'un ou de plusieurs ADNc des conjugués molécule de liaison à l'antigène/acide nucléique sur une ou plusieurs sondes de capture du réseau, dans lequel la séquence de capture d'analyte de l'ADNc se lie au domaine de capture de la sonde de capture ; et
e) la détermination (i) de la séquence du code à barres spatial ou d'un complément de celle-ci, et (ii) de la totalité ou d'une portion de la séquence de l'ADNc, ou d'un complément de celle-ci, et l'utilisation des séquences déterminées de (i) et (ii) pour identifier la molécule de liaison à l'antigène qui se lie sélectivement à l'antigène dans l'échantillon de tissu.

2. Procédé selon la revendication 1, dans lequel la détermination comprend un séquençage.

3. Procédé selon la revendication 1 ou 2, dans lequel l'étape (c) est réalisée avant (a), après (a) ou après (b).

4. Procédé selon l'une quelconque des revendications 1 à 3, dans lequel l'échantillon de tissu témoin est un échantillon de tissu sain.

5. Procédé selon l'une quelconque des revendications 1 à 4, dans lequel l'échantillon de tissu comprend une ou plusieurs régions saines et une ou plusieurs régions malades, ou dans lequel l'échantillon de tissu est un échantillon de tissu malade, facultativement dans lequel l'échantillon de tissu malade est un échantillon de tissu tumoral.

6. Procédé selon l'une quelconque des revendications 1 à 5, dans lequel le procédé comprend en outre la libération de l'ADNc à partir du conjugué molécule de liaison à l'antigène/acide nucléique avant l'étape de capture, dans lequel la libération comprend facultativement ;
(i) l'utilisation d'une RNase, facultativement dans lequel la RNase est sélectionnée dans le groupe constitué de RNase A, RNase C, RNase H, ou RNase I ; ou
(ii) la chaleur.

7. Procédé selon l'une quelconque des revendications 1 à 6, dans lequel le procédé comprend en outre :
(i) la perméabilisation de l'échantillon de tissu ;
(ii) l'imagerie de l'échantillon de tissu ; et/ou
(iii) la coloration de l'échantillon de tissu, dans lequel facultativement la coloration comprend une coloration à l'hématoxyline et à l'éosine (H&E).

8. Procédé selon l'une quelconque des revendications 1 à 7, dans lequel le réseau comprend une ou plusieurs caractéristiques, facultativement dans lequel les une ou plusieurs caractéristiques comprennent une bille.

9. Procédé selon l'une quelconque des revendications 1 à 8, dans lequel la sonde de capture comprend un domaine de clivage, un identifiant moléculaire unique (UMI), un ou plusieurs domaines fonctionnels, ou des combinaisons de ceux-ci.

10. Procédé selon l'une quelconque des revendications 1 à 9, dans lequel la molécule de liaison à l'antigène est une molécule de liaison à l'antigène simple chaîne, facultativement dans lequel la molécule de liaison à l'antigène simple chaîne comprend un fragment variable simple chaîne (scFv) ou un nanocorps.

11. Procédé selon l'une quelconque des revendications 1 à 10, dans lequel l'ADNc complémentaire de l'acide nucléique du conjugué molécule de liaison à l'antigène/acide nucléique est généré par transcription inverse.

12. Procédé selon l'une quelconque des revendications 1 à 11, dans lequel le procédé comprend la capture un de l'acide nucléique analyte or derivative thereof de l'échantillon de tissu, dans lequel l'acide nucléique se analyte lie au domaine de capture de la sonde de capture, facultativement dans lequel l'analyte d'acide nucléique, ou un dérivé de celui-ci, comprend de l'ADN ou de l'ARN.

13. Procédé selon l'une quelconque des revendications 1 à 12, dans lequel le procédé comprend en outre :
(a) la mise en contact d'une première sonde et d'une deuxième sonde avec l'échantillon de tissu, dans lequel la première sonde et la deuxième sonde comprennent chacune une ou plusieurs séquences qui sont sensiblement complémentaires de séquences d'un analyte, et dans lequel la deuxième sonde comprend un domaine de capture de sonde de capture qui est complémentaire de la totalité ou d'une portion du domaine de capture ;
(b) l'hybridation de la première sonde et de la deuxième sonde à l'analyte ;
(c) la génération d'un produit de ligature par ligature de la première sonde et de la deuxième sonde ;
(d) la libération du produit de ligature de l'analyte ;
(e) l'hybridation du produit de ligature au domaine de capture ; et
(g) la détermination (i) de tout ou partie de la séquence du produit de ligature lié au domaine de capture, ou d'un complément de celle-ci, et (ii) de la séquence du code à barres spatial, ou d'un complément de celle-ci, et l'utilisation des séquences déterminées de (i) et (ii) pour identifier l'emplacement de l'analyte dans l'échantillon de tissu.

14. Procédé selon l'une quelconque des revendications 1 à 13, dans lequel le procédé comprend en outre l'identification de l'antigène lié par le conjugué molécule de liaison à l'antigène/acide nucléique, facultativement dans lequel l'identification comprend la spectrométrie de masse, et/ou dans lequel l'identification comprend la détermination de motifs d'expression différentielle des gènes entre l'échantillon de tissu témoin et l'échantillon de tissu malade ou au sein de l'échantillon de tissu.

15. Procédé selon l'une quelconque des revendications 1 à 14, dans lequel l'échantillon de tissu est disposé sur le réseau spatial comprenant la pluralité de sondes de capture, ou dans lequel l'échantillon de tissu est disposé sur un substrat comprenant en outre facultativement l'alignement du substrat avec le réseau spatial, de telle sorte que l'au moins une portion de l'échantillon de tissu soit alignée avec l'au moins une portion du réseau spatial.
